# EUROPEAN PATENT APPLICATION

(11) **EP 4 748 847 A1**
(43) Date of publication of application: **27.05.2026**
(21) Application number: 24859952.4
(22) Date of filing: 30.08.2024
(51) Int. Cl.: C07K 7/08, A61K 38/12, A61K 47/60, A61P 1/16, A61P 13/12, A61P 17/02, A61P 43/00, C07K 7/06

(54) **PEPTIDE COMPLEX HAVING VEGFR -2 AGONIST ACTIVITY**

(30) Priority: 31.08.2023 JP 2023140983
(71) Applicant: Peptidream Inc, Kawasaki-ku Kawasaki-shi, Kanagawa 210-0821 (JP)
(72) Inventor: SUZUKI, Yoshinori, Kawasaki-shi, Kanagawa 210-0821 (JP); SHIBATA, Yoshihiro, Kawasaki-shi, Kanagawa 210-0821 (JP); KAMIKUBO, Kenta, Kawasaki-shi, Kanagawa 210-0821 (JP)
(74) Representative: Williams Powell
(86) International application number: PCT/JP2024/031157
(87) International publication number: WO 2025/047927

(57) **Abstract**

[Problem]

A new compound having VEGFR-2 agonist activity is provided.

[Solution]

A peptide complex or a pharmaceutically acceptable salt thereof comprising a first peptide and having VEGFR-2 agonist activity, wherein the first peptide has an amino acid sequence represented by X¹-W-X²-X³-X⁴-X⁵-X⁶-X⁷-X⁸-Y-X ⁹-X¹⁰-X¹¹-C (Sequence No. 1), or a peptide complex or a pharmaceutically acceptable salt thereof comprising a peptide having an amino acid sequence in which 1 to 3 amino acids are substituted, deleted, added, or inserted in the amino acid sequence shown in Sequence No. 1.

## Description

### TECHNICAL FIELD

The present invention relates to a novel peptide complex having VEGFR-2 agonist activity and to a cell culture composition comprising the peptide complex. The present invention also relates to a novel peptide complex having cell proliferative ability derived from VEGFR-2 agonist activity and to a cell culture composition comprising the peptide complex.

### BACKGROUND ART

Angiogenesis is important in normal physiological processes including embryonic development, follicular growth, and wound healing, and vascular endothelial growth factor (VEGF) is known as a principal mediator of angiogenesis. The VEGF receptor (VEGFR), which is a receptor-type tyrosine kinase, includes three types of proteins: VEGFR-1 to VEGFR-3. In particular, the binding of VEGF to VEGFR-2 induces dimerization of the receptor and autophosphorylation of tyrosine residues, and this autophosphorylation triggers the activation of several signal transduction cascades, thereby inducing endothelial cell differentiation, proliferation and migration, vascular permeability, and angiogenesis.

Studies have been reported on the use of VEGFR-2 agonists for wound healing, treatment of hepatic dysfunction and liver diseases, protection from liver injury, and treatment of kidney diseases (Patent Literatures 1, 2, 3, and 4). However, the VEGFR-2 agonists used in those studies are natural VEGF or variants thereof, and chemically synthesizable VEGFR-2 agonist peptides have not been reported.

### PRIOR LITERATURES

### PATENT LITERATURES

Patent Literature 1: Japanese Unexamined Patent Application Publication (Translation of PCT Application) No. 2008-546707
Patent Literature 2: Japanese Unexamined Patent Application Publication No. 2010-159266
Patent Literature 3: Japanese Patent No. 5111729
Patent Literature 4: Japanese Unexamined Patent Application Publication (Translation of PCT Application) No. 2009-541207

### NON-PATENT LITERATURES

Non-Patent Literature 1: Rom J Morphol Embryol 2018, 59, 455.
Non-Patent Literature 2: Biomolecules 2020, 10, 1673.
Non-Patent Literature 3: Biology Methods and Protocols, 2023, 8, bpac034.
Non-Patent Literature 4: Scientific Reports 2020, 10, 17937.
Non-Patent Literature 5: Scientific Reports 4: 6716 doi: 10.1038/srep06716.
Non-Patent Literature 6: Developmental Cell 2020, 54, 516.
Non-Patent Literature 7: Cell Reports 2018, 23, 1620.

### DISCLOSURE OF THE INVENTION

### PROBLEMS TO BE SOLVED BY THE INVENTION

One aspect of the present invention provides a novel compound having VEGFR-2 agonist activity. Another aspect of the present invention also provides a novel compound having cell proliferative ability derived from VEGFR-2 agonist activity.

Without being limited thereto, the present invention includes the following aspects.
[1] A peptide complex or a pharmaceutically acceptable salt thereof comprising a first peptide and having a VEGFR-2 agonist activity, wherein
   the first peptide has an amino acid sequence represented by X¹-W-X²-X³-X⁴-X⁵-X⁶-X⁷-X⁸-Y-X⁹-X¹⁰-X¹¹-C (SEQ ID NO: 1) or consists of an amino acid sequence in which from 1 to 3 amino acids in the amino acid sequence represented by SEQ ID NO: 1 have been substituted, deleted, added, or inserted, wherein
   X¹ is an amino acid having in a side chain a chain alkyl group optionally substituted with a polar group or an optionally substituted aromatic ring,
   X² is an amino acid having an optionally substituted alkyl group in a side chain,
   X³ is an amino acid having an optionally substituted chain alkyl group in a side chain,
   X⁴ is an amino acid having an optionally substituted alkyl group in a side chain,
   X⁵ is any amino acid,
   X⁶ is an amino acid having a chain alkyl group optionally substituted with a polar group in a side chain or is an amino acid that does not have a side chain,
   X⁷ is an amino acid having an optionally substituted chain alkyl group in a side chain,
   X⁸ is an amino acid having an optionally substituted chain alkyl group in a side chain,
   X⁹ is an amino acid having an optionally substituted aromatic ring in a side chain,
   X¹⁰ is an amino acid having an optionally substituted alkyl group in a side chain, and
   X¹¹ is any amino acid.
[2] The peptide complex or the pharmaceutically acceptable salt thereof according to [1], wherein
   X² is an amino acid having in a side chain a chain or cyclic alkyl group or a chain alkyl group optionally substituted with an aromatic ring,
   X³ is an amino acid having a chain alkyl group optionally substituted with a polar group in a side chain,
   X⁴ is an amino acid having in a side chain a chain or cyclic alkyl group or a chain alkyl group optionally substituted with a polar group,
   X⁷ is an amino acid having in a side chain a chain alkyl group optionally substituted with a polar group or a chain alkyl group optionally branched,
   X⁸ is an amino acid having in a side chain a chain alkyl group optionally substituted with a polar group or a chain alkyl group optionally branched, and
   X¹⁰ is an amino acid having in a side chain a chain or cyclic alkyl group or a chain alkyl group substituted with an aromatic ring or heterocyclic ring.
[3] The peptide complex or the pharmaceutically acceptable salt thereof according to [2], wherein
   X¹ is W, Hcit, 4Py2NH2, 3Py6NH2, F4aao, or W7N,
   X² is V, Tbg, Chg, or Hty,
   X³ is D, Q, or MetO2,
   X⁴ is V, Tbg, alT, or Chg,
   X⁵ is Q, Ahp, W, Hph, Cha, F4COO, or K,
   X⁶ is D, G, or N,
   X⁷ is D or V,
   X⁸ is L, N, or Atb,
   X⁹ is F, Bph, Yph, F4G, or F4C,
   X¹⁰ is V, Hph, Hty, Chg, or H4Py, and
   X¹¹ is D, A, S, F, F4aao, Har, or Hyp.
[4] The peptide complex or the pharmaceutically acceptable salt thereof according to [1], wherein
   X² is an amino acid having a chain or cyclic alkyl group in a side chain,
   X⁴ is an amino acid having in a side chain a chain alkyl group optionally substituted with a polar group or a chain alkyl group optionally branched,
   X⁶ is an amino acid having a chain alkyl group optionally substituted with a polar group in a side chain,
   X⁷ is an amino acid having a chain alkyl group optionally substituted with a polar group in a side chain,
   X⁸ is an amino acid having in a side chain a chain alkyl group optionally substituted with a polar group or a chain alkyl group optionally branched, and
   X¹⁰ is an amino acid having in a side chain a chain or cyclic alkyl group or a chain alkyl group optionally substituted with an aromatic ring.
[5] The peptide complex or the pharmaceutically acceptable salt thereof according to [4], wherein
   X¹ is W or W7N,
   X² is V, Tbg, or Chg,
   X³ is D,
   X⁴ is V or alT,
   X⁵ is Ahp, Cha, or K,
   X⁶ is D or N,
   X⁷ is D,
   X⁸ is L, N, or Atb,
   X⁹ is F or F4G,
   X¹⁰ is V, Hty, or Chg, and
   X¹¹ is D or S.
[6] The peptide complex or the pharmaceutically acceptable salt thereof according to [1], wherein
   the first peptide has an amino acid sequence represented by W-W-V-D-V-Q-D-D-L-Y-F-V-D-C (SEQ ID NO: 2) or consists of an amino acid sequence in which from 1 to 3 amino acids in the amino acid sequence represented by SEQ ID NO: 2 have been substituted, deleted, added, or inserted.
[7] The peptide complex or the pharmaceutically acceptable salt thereof according to [1], wherein
   the first peptide has an amino acid sequence with glycine added to a C-terminus.
[8] The peptide complex or the pharmaceutically acceptable salt thereof according to [1], wherein
   the first peptide is a peptide consisting of an amino acid sequence represented by any one of SEQ ID NOs: 3 to 51.
[9] The peptide complex or the pharmaceutically acceptable salt thereof according to [1], wherein
   the first peptide is a cyclic peptide.
[10] The peptide complex or the pharmaceutically acceptable salt thereof according to [1], wherein
   the first peptide is a cyclic peptide in which an N-terminus amino acid residue is a chloroacetylated amino acid residue, the first peptide has a cysteine residue within the peptide, and the N-terminus amino acid residue and the cysteine residue are bonded to each other.
[11] The peptide complex or the pharmaceutically acceptable salt thereof according to [1], wherein
   the peptide complex includes the first peptide, a second peptide, and a linker connecting the first peptide and the second peptide, and
   the second peptide may be the same as or different from the first peptide, and has the amino acid sequence represented by SEQ ID NO: 1 or consists of an amino acid sequence in which from 1 to 3 amino acids in the amino acid sequence represented by SEQ ID NO: 1 have been substituted, deleted, added, or inserted.
[12] The peptide complex or the pharmaceutically acceptable salt thereof according to [11], wherein
   a homology between the first peptide and the second peptide is from 90% to 100% inclusive.
[13] The peptide complex or the pharmaceutically acceptable salt thereof according to [11], wherein
   the first peptide and the second peptide have the same sequence.
[14] The peptide complex or the pharmaceutically acceptable salt thereof according to [11], wherein
   a sixth amino acid of the first peptide and a sixth amino acid of the second peptide, or a C-terminus of the first peptide and a C-terminus of the second peptide, are linked via the linker.
[15] The peptide complex or the pharmaceutically acceptable salt thereof according to [11], wherein
   the linker is a PEG linker or a linker in which from 1 to 6 amino acids are added to a PEG linker.
[16] A composition comprising the peptide complex or the pharmaceutically acceptable salt thereof according to [1] and a carrier.
[17] A composition for cell culture that is used to culture cells, the composition comprising the peptide complex or the pharmaceutically acceptable salt thereof according to [1] and a carrier.
[18] A composition for medical, diagnostic, or research use, comprising the peptide complex or the pharmaceutically acceptable salt thereof according to [1] and a carrier.

### ADVANTAGEOUS EFFECTS OF INVENTION

This invention can provide a novel compound having VEGFR-2 agonist activity. This invention can also further provide a novel compound having cell proliferation activity derived from VEGFR-2 agonist activity.

### BRIEF DESCRIPTION OF THE DRAWINGS

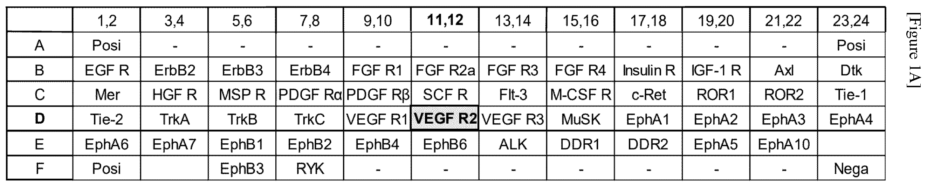

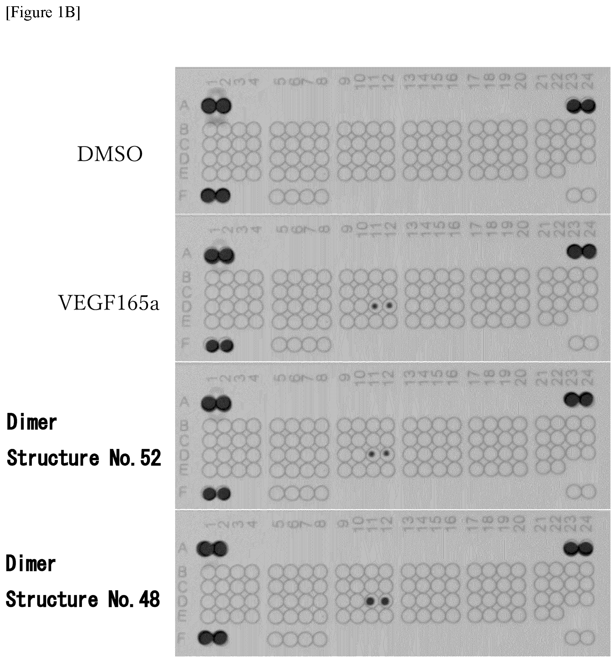

[Figure 1A] Figure 1 shows the results of the Human Phospho-RTK Array assay evaluation using the peptide complex of the present invention and VEGF165a. Figure 1A shows the array map of the target factors.
[Figure 1B] Figure 1B shows the evaluation results.

### DESCRIPTION OF EMBODIMENTS

The present invention is not limited to the following aspects. Unless otherwise stated herein, the technical and scientific terms used herein have the same meanings as those generally understood by those skilled in the art. The substances, materials, and examples disclosed herein are merely illustrative and are not intended to be limiting. Where reference is made herein to "in one aspect," this does not imply limitation to that aspect, i.e., it is non-limiting.

The first invention of this specification relates to a peptide complex or a pharmaceutically acceptable salt thereof. This peptide complex or its pharmaceutically acceptable salt comprises a first peptide and possesses VEGFR-2 agonist activity. Furthermore, this peptide complex or its pharmaceutically acceptable salt possesses cell proliferation activity derived from VEGFR-2 agonist activity. The term "pharmaceutically acceptable salt" refers to a pharmaceutically acceptable salt of the peptide complex. Pharmaceutically acceptable salts are as described below. For simplicity, the peptide complex or its pharmaceutically acceptable salt may be referred to simply as the peptide complex below.

### Peptide Complex

In one aspect, the peptide complex of the present invention comprises a first peptide and has VEGFR-2 agonist activity. In another aspect, the peptide complex of the present invention has cell proliferation activity derived from VEGFR-2 agonist activity.

The peptide complex is a peptide, peptide-containing compound, or a pharmaceutically acceptable salt thereof, comprising the first peptide and other peptides or compounds. The peptide complex may comprise one or two or more (three or more or four or more) first peptides. The peptide complex may further contain one or more partial peptides different from the first peptide. The peptide complex preferably has the first peptide and partial peptides linked via a linker. The peptide complex may be a homomultimer containing only peptides having the same amino acid sequence. The peptide complex may be a heteromeric complex containing peptides with different amino acid sequences. The peptide complex is preferably a homodimer having two first peptides with the same amino acid sequence, wherein the two first peptides are linked via a linker. In the homodimer, the two peptide moieties may have completely identical amino acid sequences or substantially identical amino acid sequences. The peptide complex exhibits VEGFR-2 agonist activity, but further, the first peptide or partial peptide constituting the peptide complex may also exhibit VEGFR-2 agonist activity. As shown in the examples, the first peptide exhibits VEGFR2 agonist activity by adopting a peptide complex structure via a linker.

In one aspect, the peptide complex of the present invention may comprise a first peptide, a second peptide, and a linker connecting the first peptide and the second peptide. In this case, the second peptide may be identical to or different from the first peptide, and it is preferable that it has the amino acid sequence shown in Sequence No. 1, or an amino acid sequence in which 1 to 3 amino acids are substituted, deleted, added, or inserted in the amino acid sequence shown in Sequence No. 1. Furthermore, the peptide complex is preferably one in which the homology between the first peptide and the second peptide is from 90% to 100% inclusive, and it is more preferably a homodimer in which the first peptide and the second peptide are substantially the same.

### First Peptide

In one aspect, the first peptide has an amino acid sequence represented by X¹-W-X²-X³-X⁴-X⁵-X⁶-X⁷-X⁸-Y-X⁹-X¹⁰-X¹¹-C (Sequence No. 1), or comprises an amino acid sequence in which 1 to 3 (1, 2, or 3) amino acids are substituted, deleted, added, or inserted in the amino acid sequence shown in Sequence No. 1. The substituted amino acids include pharmaceutically acceptable salts of the amino acids. Preferred examples of peptides having a sequence consisting of an amino acid sequence in which an amino acid is substituted, deleted, added, or inserted in the amino acid sequence indicated by Sequence No. 1 are peptides having an amino acid sequence in which a conservative amino acid substitution has been made.

### Conservative amino acid substitution

When 1, 2, or 3 amino acid residues are substituted, deleted, added, or inserted from a particular sequence, conservative amino acid substitutions are preferably performed. "Conservative amino acid substitution" means substitution with a functionally equivalent or similar amino acid. Conservative amino acid substitution in a peptide causes static changes in the amino acid sequence of the peptide. For example, 1 or more amino acids having similar polarities act in a functionally equivalent manner to cause a static change in the amino acid sequence of the peptide. In general, substitution within the group can be considered conservative in structure and function. However, as is obvious to those skilled in the art, the role played by a particular amino acid residue can be determined by its implications in the three-dimensional structure of the molecule comprising that amino acid. For example, a cysteine residue can take a less polar, oxidized (disulfide) form compared to the reduced (thiol) form. The long aliphatic moiety of an arginine side chain can constitute a structurally and functionally important feature. A side chain comprising an aromatic ring (such as tryptophan, tyrosine, and phenylalanine) can also contribute to ion-aromatic or cation-pi interactions. In such a case, the substitution of an amino acid having these side chains with an amino acid belonging to an acidic or non-polar group can be structurally and functionally conservative. Residues of proline, glycine, cysteine (in disulfide form), and the like can have a direct effect on the steric structure of the main chain and often cannot be substituted without structural distortion.

Conservative amino acid substitution includes specific substitutions based on the side chain similarity (L. Lehninger, Biochemistry, 2nd edition, pp. 73-75, Worth Publishers, New York (1975)) and typical substitutions, as shown below.

Conservative amino acid substitution is preferably, for example, substitution to another amino acid belonging to the same group to which an amino acid belongs in the group of natural amino acids classified based on their common side-chain properties, as follows.

Hydrophobic (also referred to as non-polar) amino acids: Hydrophobic amino acids are amino acids that are hydrophobic (non-polar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").
The hydrophobic amino acids can also be further classified into the following groups.
Aliphatic amino acids: Aliphatic amino acids are amino acids having a fatty acid or hydrogen in a side chain, and include Ala, Gly, Val, Ile, and Leu.
Aliphatic, branched amino acids: Aliphatic, branched amino acids are amino acids having a branched fatty acid in a side chain and include Val, Ile, and Leu.
Aromatic amino acids: Aromatic amino acids are amino acids having an aromatic ring in a side chain and include Trp, Tyr, and Phe.

Hydrophilic (also referred to as polar) amino acids: Hydrophilic amino acids are amino acids that are hydrophilic (polar) and include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acids can also be further classified into the following groups.

Acidic amino acids: Acidic amino acids are amino acids with an acidic side chain and include Asp and Glu.

Basic amino acids: Basic amino acids are amino acids with a basic side chain and include Lys, Arg, and His.

Neutral amino acids: Neutral amino acids are amino acids with a neutral side chain and include Ser, Thr, Asn, Gln, and Cys.

Gly and Pro can be also classified as "amino acids which affect the direction of the main chain," and amino acids having a sulfur molecule in a side chain, such as Cys and Met, can be classified as "sulfur-containing amino acids."

Herein, "amino acids" include not only natural amino acids but also non-natural amino acids. Examples of non-natural amino acids include N-alkyl amino acids in which the natural amino acids above are N-alkylated, and amino acids in which nitrogen that forms a peptide bond is modified with a branched or unbranched lower (e.g., C1 to C5, preferably C1 to C3, or more preferably C1) alkyl group. Among the N-alkyl amino acids, N-ethyl amino acids, N-butyl amino acids, and N-methyl amino acids are preferable, and N-methyl amino acids are more preferable. Non-natural amino acids include chemically modified amino acids such as D-amino acids, β-amino acids, γ-amino acids, amino acid variants, and amino acid derivatives; and amino acids which are not constituent materials of proteins in vivo such as norleucine and ornithine. Non-natural amino acids further include amino acids in which a functional group is further added to a side chain of a natural amino acid or is substituted with another functional group (such as an amino acid with substitutions or additions in an arylene group moiety or alkylene group moiety of a side chain, an amino acid with increased carbon atoms in the arylene group, alkylene group, or alkyl group of a side chain, an amino acid with substitutions in an aromatic ring of a side chain, or a heterocyclized or condensed and cyclized amino acid).

The addition or substitution of a functional group or other structure to a side chain of a natural amino acid can impart properties different from those of the natural amino acid. For example, A4p is alanine with a piperidyl group attached to a side chain, wherein the addition of the piperidyl group makes A4p basic and polar, unlike alanine, which belongs to the non-polar amino acid group.

In other words, a non-natural amino acid having similar side-chain properties can be included in the group of natural amino acids above based on their common side-chain properties. For example, N-methylarginine (MeR), which is an N-methylated amino acid of arginine belonging to the basic amino acid group, is a non-natural amino acid but can be classified as a basic amino acid because it exhibits basic properties. Thus, non-natural amino acids that exhibit similar side-chain properties to amino acids can also be included as targets for conservative amino acid substitution.

Non-limiting examples of non-natural amino acids include N-methyl amino acids, 4Py, alT, Cit, P4Sh, F4CON, F4COO, 3Py, HyP, SMe, A4paa, Atp, Hgl, KAc, Nal1, W6N, W7N, and PeG. N-methyl amino acids can be classified into N-alkyl amino acids or classified according to the side-chain properties of their non-N-methylated original amino acid.

### Peptide length

The first peptide may include, for example, the amino acid sequences represented by SEQ ID NOs: 2 to 51 or an amino acid sequence of positions 1 to 14 of the amino acid sequence and an added amino acid residue. The added amino acid residue may be included in a peptide forming a cyclic structure or may be further added in a linker-like manner from the cyclic peptide. Although the number of amide bonds (the number and length of the amino acids) in the peptide and the peptide moiety is not particularly limited, the total number of amino acid residues (referring to the number of amino acid residues included in the peptide forming a cyclic structure; when amino acid residues are added in a linker-line manner from the cyclic peptide, these amino acids are not included) is preferably within 20 residues. The peptide length, in terms of amino acid residues, is preferably 6 or more, 7 or more, 8 or more, 9 or more, 10 or more, or 11 or more and preferably 19 or less or 18 or less. More preferably, the peptide length, in terms of amino acid residues, is from 13 to 16 inclusive, and most preferably, is 14 or 15.

A preferred example of the first peptide is a peptide having an amino acid sequence represented by X¹-W-X²-X³-X⁴-X⁵-X⁶-X⁷-X⁸-X-X⁹-X¹⁰-X¹¹-C (SEQ ID NO: 1), wherein
X¹ is an amino acid having in a side chain a chain alkyl group optionally substituted with a polar group or an optionally substituted aromatic ring,
X² is an amino acid having an optionally substituted alkyl group in a side chain,
X³ is an amino acid having an optionally substituted chain alkyl group in a side chain,
X⁴ is an amino acid having an optionally substituted alkyl group in a side chain,
X⁵ is any amino acid,
X⁶ is an amino acid having a chain alkyl group optionally substituted with a polar group in a side chain or is an amino acid that does not have a side chain,
X⁷ is an amino acid having an optionally substituted chain alkyl group in a side chain,
X⁸ is an amino acid having an optionally substituted chain alkyl group in a side chain,
X⁹ is an amino acid having an optionally substituted aromatic ring in a side chain,
X¹⁰ is an amino acid having an optionally substituted alkyl group in a side chain, and
X¹¹ is any amino acid.

Another preferred example of the first peptide is a peptide having an amino acid sequence represented by SEQ ID NO: 1, wherein
X² is an amino acid having in a side chain a chain or cyclic alkyl group or a chain alkyl group optionally substituted with an aromatic ring,
X³ is an amino acid having a chain alkyl group optionally substituted with a polar group in a side chain,
X⁴ is an amino acid having in a side chain a chain or cyclic alkyl group or a chain alkyl group optionally substituted with a polar group,
X⁷ is an amino acid having in a side chain a chain alkyl group optionally substituted with a polar group or a chain alkyl group optionally branched,
X⁸ is an amino acid having in a side chain a chain alkyl group optionally substituted with a polar group or a chain alkyl group optionally branched, and
X¹⁰ is an amino acid having in a side chain a chain or cyclic alkyl group or a chain alkyl group substituted with an aromatic ring or heterocyclic ring.

Another preferred example of the first peptide is a peptide having an amino acid sequence represented by SEQ ID NO: 1, wherein
X¹ is W, Hcit, 4Py2NH2, 3Py6NH2, F4aao, or W7N,
X² is V, Tbg, Chg, or Hty,
X³ is D, Q, or MetO2,
X⁴ is V, Tbg, alT, or Chg,
X⁵ is Q, Ahp, W, Hph, Cha, F4COO, or K,
X⁶ is D, G, or N,
X⁷ is D or V,
X⁸ is L, N, or Atb,
X⁹ is F, Bph, Yph, F4G, or F4C,
X¹⁰ is V, Hph, Hty, Chg, or H4Py, and
X¹¹ is D, A, S, F, F4aao, Har, or Hyp.

Another preferred example of the first peptide is a peptide having an amino acid sequence represented by SEQ ID NO: 1, wherein
X² is an amino acid having a chain or cyclic alkyl group in a side chain,
X⁴ is an amino acid having in a side chain a chain alkyl group optionally substituted with a polar group or a chain alkyl group optionally branched,
X⁶ is an amino acid having a chain alkyl group optionally substituted with a polar group in a side chain,
X⁷ is an amino acid having a chain alkyl group optionally substituted with a polar group in a side chain,
X⁸ is an amino acid having in a side chain a chain alkyl group optionally substituted with a polar group or a chain alkyl group optionally branched, and
X¹⁰ is an amino acid having in a side chain a chain or cyclic alkyl group or a chain alkyl group optionally substituted with an aromatic ring or heterocyclic ring. Complexes containing these peptides have been shown, in the Examples, to possess VEGFR-2 agonist activity as well as cell-proliferative ability.

Another preferred example of the first peptide is a peptide having an amino acid sequence represented by SEQ ID NO: 1, wherein
X¹ is W or W7N,
X² is V, Tbg, or Chg,
X³ is D,
X⁴ is V or alT,
X⁵ is Ahp, Cha, or K,
X⁶ is D or N,
X⁷ is D,
X⁸ is L, N, or Atb,
X⁹ is F or F4G,
X¹⁰ is V, Hty, or Chg, and
X¹¹ is D or S.

In this specification, "may be substituted" means that it is unsubstituted or substituted with any number of any substituents.

Amino acids having an alkyl group in the side chain that may be substituted "Amino acids having an alkyl group in the side chain that may be substituted" means amino acids having an alkyl group in the side chain, for example, but not limited to, alanine, proline, isoleucine, leucine, valine, and the like, wherein the alkyl group may be either chain-shaped or cyclic, and further means amino acids wherein the side chain bearing the alkyl group is further substituted with substituents such as alkyl groups, aromatic rings, heterocyclic rings, hydroxyl groups, and the like, or is unsubstituted.

Amino acids having a chain alkyl group in the side chain, which may be substituted "Amino acids having a chain alkyl group in the side chain, which may be substituted" means amino acids having a chain alkyl group in the side chain, for example, but not limited to, alanine, isoleucine, leucine, valine, etc., wherein the side chain of the amino acid having a chain alkyl group in the side chain is further substituted with substituents such as alkyl groups, polar groups, and the like, for example, but not limited to, carboxyl groups, amide groups, methanesulfonyl groups, and the like, or is unsubstituted.

Amino acids having a chain alkyl group that may be substituted with a polar group in the side chain
"Amino acids having a chain alkyl group that may be substituted with a polar group in the side chain" means amino acids having the aforementioned chain alkyl group in the side chain, wherein the side chain of the amino acid having the chain alkyl group is further substituted with a polar group, for example, but not limited to, a urea group, an amino group, a carboxyl group, an amide group, a hydroxyl group, a methanesulfonyl group, and the like, or amino acids whose side chains are not substituted with polar groups, but preferably the former.

Amino acids lacking a side chain
"Amino acids lacking a side chain" means glycine or glycine derivatives.

Amino acids having a linear or cyclic alkyl group in the side chain
"Amino acids having a linear or cyclic alkyl group in the side chain" means amino acids having the aforementioned alkyl group in the side chain, wherein the alkyl group may be either linear or cyclic.

Amino acids having a chain alkyl group that may be substituted with an aromatic ring in the side chain
"Amino acids having a chain alkyl group in the side chain, which may be substituted with an aromatic ring" means amino acids having the aforementioned chain alkyl group in the side chain, wherein the side chain of the amino acid having the chain alkyl group may be further substituted with an aromatic ring or may not be substituted with an aromatic ring, but preferably the former.

Amino acids having a chain alkyl group optionally branched in the side chain
"Amino acids having a chain alkyl group optionally branched in the side chain" are amino acids having the aforementioned chain alkyl group in the side chain, wherein the side chains of these amino acids may further be branched. Amino acids having a chain alkyl group optionally branched in the side chain are preferably amino acids having a branched chain alkyl group.

Amino acids having an aromatic ring that may be substituted in the side chain
"Amino acids having an aromatic ring that may be substituted in the side chain" means amino acids having an aromatic ring in the side chain, wherein the carbon atoms constituting said aromatic ring or the functional groups bonded to said aromatic ring may be substituted or unsubstituted, preferably the former. The aromatic ring that may be substituted in the side chain may have a fused ring structure, such as a benzene ring, an indole ring, or a naphthalene ring, and may have some of its carbon atoms (C) substituted with other atoms such as nitrogen (N) or other molecules. The aromatic ring may also be a heterocyclic ring. Furthermore, amino acids having an aromatic ring that may be substituted on the side chain may include, for example, amino acids such as tyrosine where the hydroxyl group of the side chain is substituted with another functional group. The type and position of the substituted functional group are not particularly limited and may be selected arbitrarily, such as alkyl, phenyl, cycloalkyl, hydroxyl, halogen, and the like. Amino acids having an aromatic ring that may be substituted on the side chain include, for example, tyrosine, tryptophan, histidine, phenylalanine, and the like. Furthermore, amino acids having an aromatic ring that may be substituted on the side chain may be non-natural amino acids, such as 4Py2NH2, 3Py6NH2, F4aao, W7N, Bph, Yph, F4G, F4C, and the like.

Amino acids having a chain alkyl group substituted with an aromatic ring or a heterocyclic ring in the side chain
"Amino acids having a chain alkyl group substituted with an aromatic ring or a heterocyclic ring in the side chain" means amino acids having the aforementioned chain alkyl group in the side chain, wherein the side chains of these amino acids are further substituted with aromatic rings or heterocyclic rings.

Preferred examples of the first peptide also have the amino acid sequence shown as W-W-V-D-V-Q-D-D-L-Y-F-V-D-C (Sequence No. 2), or are peptides consisting of an amino acid sequence in which 1 to 3 amino acids are substituted, deletion, addition, or insertion in the amino acid sequence shown in Sequence No. 2. When an amino acid is substituted or deleted, the position of that amino acid is preferably one of the 1st, 3rd, 4th, 5th, 6th, 7th, 8th, 9th, 11th, 12th, or 13th positions in the amino acid sequence of Sequence No. 2.

Preferred examples of the first peptide are peptides having an amino acid sequence with an additional glycine added at the C-terminus. Furthermore, in peptides of Sequence Nos. 14-35 and Sequence Nos. 37-51, it is preferable that the carboxyl group at the C-terminus of the 15th glycine is converted to an amide.

A preferred example of the first peptide is a peptide consisting of an amino acid sequence represented by any one of SEQ ID NOs: 2 to 51 or a pharmaceutically acceptable salt thereof. Examples of the pharmaceutically acceptable salt include inorganic acid salts, organic acid salts, inorganic base salts, organic base salts, and acidic or basic amino acid salts. Examples of the inorganic acid salts include hydrochlorides, hydrobromides, sulfates, nitrates, and phosphates. Examples of the organic acid salts include acetates, succinates, fumarates, maleates, tartrates, citrates, lactates, stearates, benzoates, methanesulfonates, and p-toluenesulfonates. Examples of the inorganic base salts include alkali metal salts such as sodium salts and potassium salts; alkali earth metal salts such as calcium salts and magnesium salts; aluminum salts; and ammonium salts. Examples of the organic base salts include diethylamine salts, diethanolamine salts, meglumine salts, and N,N'-dibenzylethylenediamine salts. Examples of the acidic amino acid salts include aspartates and glutamates. Examples of the basic amino acid salts include arginine salts, lysine salts, and ornithine salts. The first peptide and the complex may be pharmaceutically acceptable salts or solvates. An example of the solvate is a hydrate.

A preferred example of the first peptide is a cyclic peptide. A preferred example of the first peptide is a cyclic peptide wherein the N-terminus amino acid residue is a chloroacetylated amino acid residue, the peptide contains a cysteine residue, and the N-terminus amino acid residue and the cysteine residue are bonded together.

### Cyclic peptide

The cyclic peptide refers to a peptide wherein two amino acids are bonded to form a ring in whole or in part. In the present application, the cyclic peptide also encompasses peptides wherein the amino acids form a cross-linked structure, peptides wherein a cyclic structure is formed by a lactam ring formation or macrocyclization reaction, and peptides having a lasso peptide-like structure. In other words, in the present application, the cyclic peptide may have a linear chain portion as long as a portion of the peptide forms a cyclic structure.

In the present specification, some amino acids may be modified for the cyclization of the peptide. The peptide also encompasses peptides comprising such modified amino acids. An example of a modification for cyclization is the addition of a chloroacetyl group to an amino acid at the N-terminus, which binds to a cysteine residue in the peptide to form a ring. The peptide of the present application also encompasses peptides comprising various (natural/non-natural) amino acids with added chloroacetyl groups.

Peptides generally have poor metabolic stability in vivo and are large in size, which makes it difficult for the peptides to permeate cell membranes. In order to address this problem, a method that cyclizes the peptides has been used. It has been suggested that the cyclization of the peptides improves protease resistance, improves metabolic stability, and limits conformational changes, thereby increasing rigidity and improving membrane permeability and affinity with target proteins.

The cyclization of the peptides can be performed according to known methods. Although not limited to this, for example, by designing the peptide such that it comprises two or more cysteine residues, a cyclic structure can be formed by a disulfide bond after translation. The cyclization can also be achieved by synthesizing a peptide having a chloroacetyl group at the N-terminus and positioning cysteine residues in the peptide using a genetic code reprogramming technique according to the method of Goto et al (Y. Goto, et al. ACS Chem.Biol.3 120-129 (2008)). This causes the mercapto group to do a spontaneous nucleophilic attack on the chloroacetyl group after translation, and the peptide is cyclized by a thioether bond. Other combinations of amino acids that bond to form rings may be positioned within the peptide to achieve cyclization by the genetic code reprogramming technique. Alternatively, a peptide having a cyclamide at the N-terminus may be synthesized and cyclized by positioning an L-2-aminoadipic acid residue in the peptide and creating a bond between them. Thus, the cyclization method is not particularly limited and any known cyclization method can be used.

### Linker

In one aspect, the first peptide or the partial peptide may have an added linker. The linker may have a structure in which a plurality of peptide-containing compounds are linked to each other in the peptide-containing complex. Examples of the linker include an amino acid linker (a peptide linker), a chemical linker, a fatty acid linker, a nucleic acid linker, and a glycan linker, and the linker may be a complex of the chemical linker, the peptide linker, and the like. An example of the chemical linker is a polyethylene glycol (PEG) linker. The PEG linker may be a linker consisting of 1 to 36 ethylene glycol units. The linker may also be a fatty acid linker comprising a divalent chemical moiety derived from a fatty acid. The amino acid (peptide) linker is a linker comprising at least one amino acid, and, for example, a glycine-rich peptide linker of a peptide such as one having the sequence [Gly-Gly-Gly-Gly-Ser]n (wherein n is 1, 2, 3, 4, 5, or 6), similar to those described in U.S. Patent No. 7,271,149, and the serine-rich peptide linker described in U.S. Patent No. 5,525,491 can be used. Although not limited to the following, the addition of the linker may change the physical properties (e.g., solubility) of the peptide. In addition, a combination of the linkers above may be suitable. For example, Gly-Lys may be bonded as an amino acid linker, and a PEG linker may be bonded to the terminus of the side chain of Lys. The linker may also have a structure wherein the amino acids and PEGs are bonded alternately, such as PEG-amino acid-PEG. Here, PEG denotes a PEG linker. Another example of a linker is one in which 1 to 6 amino acids are attached to the PEG linker portion. This linker may have a structure in which amino acids are attached to one end of the PEG linker portion, or it may have a structure in which amino acids are attached to both ends of the PEG linker portion. A preferred example of the amino acid attached to the PEG portion is K (Lys), but it is not limited to K and may be other amino acids. Preferred examples of the linker are linkers having the structures shown in Table 3.

The linker may be added at any position. For example, the linker may be located on the C-terminus side of the peptide, bonded to Cys, which forms a cyclic structure by binding to the amino acid at the 1st position, or it may be bonded to an amino acid in the cyclic peptide. Although not limiting, the linker is preferably bonded to the Cys located at the C-terminus or to the side chain of an amino acid contained in the cyclic peptide. More preferably, the linker is bonded to the Cys located at the C-terminus or to the side chain of the sixth amino acid contained in the cyclic peptide.

For example, in the amino acid sequences represented by SEQ ID NOs: 2 to 51, the Gly at the 15th position can be regarded as a linker. Although not limited to the following, for example, a cyclic peptide wherein the first amino acid of the amino acid sequence represented by sequence numbers 2 to 51 is bound to the 14th amino acid, Cys, and wherein two cyclic peptide structures have glycine further attached to the 14th Cys, wherein the dimer structure is formed via the structure shown in Table 2 from said added glycine, the linker structure may be the structure shown in Table 2, or it may be considered a structure wherein the structure shown in Table 2 is bonded to the glycine. The dimer may be one where the sixth amino acid of the first peptide and the sixth amino acid of the second peptide are bonded via the linker, or one where the C-terminus of the first peptide and the C-terminus of the second peptide are bonded via the linker. Among these, the former is preferred.

### VEGFR-2

VEGFR-2 is a member of the tyrosine kinase receptor family, also known as KDR (kinase insert domain receptor) or Flk-1 (fetal liver kinase 1). VEGFR-2 is highly expressed in vascular endothelial cells and lymphatic vessels, and among the VEGF ligands, it has a higher affinity for VEGF-A and VEGF-E, while having a lower affinity for VEGF-C and VEGF-D. (Non-Patent Literature 1)

Like other tyrosine kinase receptors, VEGFR-2 possesses a tyrosine kinase domain within the cell. When VEGF binds to the extracellular domain of VEGFR-2, autophosphorylation of tyrosine residues occurs. Through the activation of several signaling pathways and mechanisms, this induces vasculogenesis and non-pathological or pathological angiogenesis. (Non-Patent Literature 1)

On the other hand, VEGF-A is a crucial ligand for angiogenesis, playing a significant role in hemangiogenesis and vascular growth. VEGF-A binds to both VEGFR-1 and VEGFR-2. The binding affinity of VEGF-A for VEGFR-2 is lower than its affinity for VEGFR-1, but the tyrosine kinase activity of VEGFR-2 is higher than that of VEGFR-1. Therefore, considering these factors comprehensively, VEGFR-2 is considered the primary signaling receptor for VEGF-A binding. (Non-Patent Literature 1, 2)

### VEGFR-2 agonist activity

In one aspect, the peptide complex of the present invention preferably has VEGFR-2 agonist activity.

VEGFR-2 agonist activity refers to the ability to produce effects similar to those exerted by VEGF, a naturally occurring VEGFR-2 agonist, on VEGFR-2. Specifically, it refers to the ability to VEGFR-2-specifically activate VEGFR-2 dimerization and signaling pathways.

"VEGFR-2 agonist activity" may be evaluated, for example, using kinase assay systems employing VEGFR-2 as the target protein. For example, non-limiting examples include evaluation using AlphaLISA assays or NFAT assays that detect phosphorylation of VEGFR-2 or ERK. Non-limiting examples include evaluation using the AlphaLISA^{®} SureFire^{®} Ultra VEGFR-2 (p-Tyr1175) Assay Kit (PerkinElmer), AlphaLISA^{®} SureFire^{®} Ultra p-ERK1/2 (T202/Y204) Kit (PerkinElmer), VEGFR2/NFAT Reporter-HEK293 Recombinant Cell line (BPS Bioscience) and VEGF Bioassay Kit (Promega). In any of these assay systems, a peptide or peptide complex is considered to possess VEGFR-2 agonist activity when it detects VEGFR-2 agonist activity upon use at the optimal concentration under optimal conditions following standard procedures. Non-limiting examples include demonstrating a peptide's agonist activity via its EC50 value.

### Nucleic acid

This specification also provides a nucleic acid which encodes the first peptide and the peptide complex. Herein, the "nucleic acid" may be a natural or non-natural nucleic acid, including DNA, RNA, and chimeras thereof, but is not limited thereto. The nucleic acid can be designed and produced by a known method based on the sequences of the first peptide and the peptide complex.

### Peptide-drug conjugate (PDC)

In one aspect, the present invention relates to a complex (peptide drug conjugate) comprising a peptide or peptide complex of the present invention, a desired substance to be delivered to VEGFR-2, and a linker for binding said peptide or peptide complex to said substance. The peptide complex is expected to bind to VEGFR-2 due to its VEGFR-2 agonist activity. Therefore, the peptide or peptide complex is capable of delivering the substance to VEGFR-2.

The substance may be any substance desired by those skilled in the art that is desired to be delivered to VEGFR-2. Examples of the substance include, but are not limited to, the following:
Compounds: May be low-molecular-weight compounds and medium-molecular-weight compounds, and examples thereof include known low-molecular-weight pharmaceutical agents.
Peptides: May be a peptide that binds to a target in the body and exhibits some effect. For example, the peptide may be a cyclic peptide.
RIs: May be any compound labeled with a radioisotope, such as a radioisotope-labeled low- or medium-molecular-weight compound or antibody. Examples of RIs include compounds for PET scans.
Proteins: May be any protein that exhibits a useful function in the body, such as an antibody or enzyme. Examples of proteins include enzymes used in enzyme replacement therapy.
Nucleic acids: May be DNA, RNA, or chimeras thereof, and are not particularly limited. Examples include nucleic acid pharmaceuticals.

Molecules used in drug delivery systems (DDS): May be a known molecule used in a DDS such as a liposome or micelle. The DDS molecule may further contain a compound such as a pharmaceutical inside.

The substance desired to be transported to VEGFR-2 may be a complex of the substances listed above.

### Composition

In one aspect, the present invention relates to a composition comprising the peptide complex and a carrier. An example of the carrier is a mixture of one or more of water such as sterile water, pure water, or distilled water; a physiological saline solution; a glucose solution; an alcohol such as ethanol; a polyalcohol such as glycerol, propylene glycol, or polyethylene glycol; a sterile organic solvent; a water-soluble starch; and PBS.

### Cell culture composition

In one aspect, the present invention relates to a cell culture composition. The cell culture composition is a composition used for cell culture. The peptide complex, possessing VEGFR-2 agonist activity, can also be used as a medium reagent or additive for cell culture, preferably for mammalian cells, and more preferably for human cells. The aforementioned medium reagents and additives for cell culture may also be medium reagents and additives for culturing cells for the production of cultured meat.

Furthermore, the aforementioned peptide complex can also be used as a reagent or additive in a medium for producing liver organoids for use in the treatment of liver disease. The liver organoids are created, non-exclusively, through co-culture of hepatodermal cells, vascular endothelial cells, and mesenchymal cells derived from pluripotent stem cells. The peptide complex may be used in a differentiation induction system for vascular endothelial cells (Non-Patent Literature 3, 4), but is not limited thereto. VEGF, a naturally occurring VEGFR-2 agonist, induces differentiation of pluripotent stem cells into vascular endothelial cells and vascular wall cells. Co-culturing these with cardiomyocytes, intestinal epithelial cells, pancreatic islets, and the like, contributes to the formation of higher-order structures and the establishment and maintenance of organoids (Non-Patent Literature 5, 6, 7).
Given that VEGF is a fundamental molecule in tissue generation, the aforementioned peptide complex can be utilized as a reagent or additive for producing the various cells described above.

The medium is not particularly limited as long as it is a medium for culturing cells or tissues. The medium may be a serum-containing medium, or preferably a serum-free or low-serum medium.

In one aspect, the culture medium additive may be in a solution form or a dried solid form (e.g., solid, powder, or the like). If the culture medium additive is in the solution form, it may be used as it is as a culture medium, or it may be diluted using a solvent, mixed with the additive above as needed, and then used as a culture medium. Examples of the solvent used for the dilution include water, a buffer solution, a saline solution, and media for culturing various cells and tissues, which may be used alone or in a combination of 2 or more of them.

If the culture medium additive is in the dried solid form, it may be dissolved in a solvent such as water, a buffer solution, a saline solution, and media for culturing various cells and tissues, mixed with the additive above as needed, and then used as a culture medium.

The content of the peptide complex of the present invention in the medium for culturing cells or tissues or in the medium for cells obtained therefrom is, for example, as a final concentration relative to the total amount of the composition or the medium, about 0.01 nmol/L to about 10000 nmol/L, preferably about 0.1 nmol/L to about 1000 nmol/L, more preferably about 0.5 nmol/L to about 1000 nmol/L, or even more preferably about 1 nmol/L to about 100 nmol/L.

### Medical Composition

In one aspect, the present invention relates to a medical composition. The medical composition, in one aspect, comprises a peptide complex of the present invention, a pharmaceutically acceptable salt or solvate thereof (hereinafter referred to simply as "peptide" for simplicity). The medical composition preferably contains an effective amount of the peptide complex as an active ingredient.

Diseases targeted by the medical composition include any disease caused by, exacerbated by, or otherwise associated with an increase or decrease in VEGFR-2 expression or activity, or any disorder caused by, exacerbated by, or otherwise associated with a decrease in VEGF signaling or any other intracellular signaling cascade activated via VEGFR-2.

Examples of wounds include chronic wounds, acute wounds, and normal wounds. Chronic wounds refer to wounds that do not heal, including but not limited to arterial ulcers, diabetic ulcers, pressure ulcers, and venous ulcers. Acute wounds can develop into chronic wounds. Acute wounds include, but are not limited to, those caused by thermal injury, trauma, surgery, excision of extensive skin cancer, deep fungal and bacterial infections, vasculitis, scleroderma, pemphigus, and toxic epidermal necrolysis. Normal wounds refer to wounds undergoing normal wound healing and repair.

Pathological conditions of liver dysfunction or liver disease relate to any structural and/or functional abnormality of the liver, including but not limited to liver failure, hepatitis, cirrhosis, toxic liver injury, drug-induced liver injury, hepatic encephalopathy, hepatic coma, and hepatic necrosis. Protection from liver damage refers to protection against structural or functional liver injury arising directly or indirectly from internal or external factors (e.g., chemical agents, biological agents, and the like), or a combination thereof.

Kidney diseases include, but are not limited to, inflammatory kidney diseases, nephritis, glomerulosclerosis, glomerulonephritis, and focal segmental glomerulosclerosis. They also include infections that cause kidney disease.

The route of administration for the pharmaceutical composition above is not particularly limited, and the pharmaceutical composition may be administered orally or parenterally. Examples of parenteral administration include administration by injection such as by intramuscular injections, intravenous injections, or subcutaneous injections; transdermal administration; and transmucosal (transnasal, oral, ocular, pulmonary, vaginal, or rectal) administration.

The peptides in the pharmaceutical composition can be modified in various ways in view of their metabolic and excretory properties. For example, polyethylene glycol (PEG) or a glycan can be added to polypeptides to increase their retention time in blood and decrease their antigenicity. In addition, biodegradable polymer compounds such as poly(lactic-co-glycolic acid) (PLGA); porous hydroxyapatite; liposomes; surface-modified liposomes; and emulsions, nanoparticles, nanospheres, and the like prepared from unsaturated fatty acids may be used as a sustained release substrate, and the polypeptide may be encapsulated therein. When the pharmaceutical composition is administered transdermally, a weak electric current can be applied to the surface of the skin to permeate the stratum corneum (iontophoresis method).

The active ingredients of the pharmaceutical composition may be used as they are, or a pharmaceutically acceptable carrier, excipient, additive, or the like may be added to the active ingredients to formulate the pharmaceutical composition. Examples of the dosage form include a liquid (e.g., an injection), a dispersant, a suspension, a tablet, a pill, a powder, a suppository, a powdered medicine, fine granules, granules, a capsule, a syrup, a troche, an inhalant, an ointment, eye drops, nose drops, ear drops, a gel patch, and the like.

The formulation may be performed through conventional means using, for example, an excipient, a binder, a disintegrant, a lubricant, a solvent, a solubilizer, a colorant, a flavor or odor masking agent, a stabilizing agent, an emulsifier, a sorbefacient, a surfactant, a pH adjuster, a preservative, an antioxidant, and the like, as appropriate.

Examples of components used for the formulation include, but are not limited to, purified water; a saline solution; a phosphate buffer solution; dextrose; glycerol; pharmaceutically acceptable organic solvents such as ethanol; animal and vegetable oils; lactose; mannitol, glucose, sorbitol, crystalline cellulose, hydroxypropyl cellulose, starch, cornstarch, silicic anhydride, aluminum magnesium silicate, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, human serum albumin, trehalose, and polysorbate, etc..

The aforementioned absorption enhancer can be used to improve the absorption of poorly absorbed drugs. The sorbefacient may be a surfactant such as polyoxyethylene lauryl ethers, sodium lauryl sulfate, saponin, and the like; a bile salt such as glycocholate, deoxycholate, taurocholate, and the like; a chelating agent such as EDTA and salicylic acids; fatty acids such as caproic acid, capric acid, lauric acid, oleic acid, linoleic acid, or mixed micelles; enamine derivatives; N-acyl collagen peptides; N-acyl amino acids; cyclodextrins; chitosans; and nitric oxide donors.

The pill or tablet may also be coated with a saccharide or substance for gastric or enteric coating. The injection may contain distilled water for injections, a saline solution, propylene glycol, polyethylene glycol, a vegetable oil, alcohols, and the like. The injection may further contain a humectant, an emulsifier, a dispersant, a stabilizing agent, a solvent, a solubilizer, a preservative, and the like.

In one aspect, the pharmaceutical composition of the present invention may be administered in combination with other pharmaceuticals or treatments useful for the disorders above.

When the pharmaceutical composition of the present invention is administered to mammals (e.g., humans, mice, rats, guinea pigs, rabbits, dogs, horses, monkeys, pigs, sheep, and the like), especially humans, the dose depends on the symptoms, age, sex, weight, and sensitivity differences of the subject, administration method, administration interval, types of active ingredients, and type of formulation. The dose is not particularly limited, but amounts of, for example, 30 µg to 1000 mg, 100 µg to 500 mg, or 100 µg to 100 mg may be administered for 1 or several doses. For administration by injection, 1 µg/kg to 3000 µg/kg or 3 µg/kg to 1000 µg/kg may be administered for 1 or several doses, depending on the weight of the subject.

### Diagnostic Composition

In one aspect, the present invention relates to a diagnostic composition. Since the peptide complex possesses VEGFR-2 agonist activity, it is considered to bind to VEGFR-2. Therefore, it can also be used as a diagnostic reagent for detecting VEGFR-2. As such a diagnostic reagent, it may be a detection reagent that detects the expression level of VEGFR-2. When used as a detection reagent, the peptide complex of the present invention may be labeled to be detectable. In this manner, the peptide complex or compositions containing it can be used as a diagnostic reagent for detecting VEGFR-2.

### Research Composition

In one aspect, the present invention relates to a research composition. Since the peptide complex exhibits VEGFR-2 agonist activity, it is considered to bind to VEGFR-2. Therefore, it can be favorably used in research involving VEGFR-2.

In one aspect, the present invention relates to a complex comprising a peptide and/or a substance further conjugated to a peptide and a linker, wherein the complex is tested for at least one of:
a) solubility in a solvent,
b) binding ability to VEGFR-2,
c) toxicity to cells and/or tissues, and
d) toxicity to experimental animals,
wherein the peptide and/or complex has an amino acid sequence in which 1 to 3 amino acid residues are deleted, substituted, inserted, and/or added to the amino acid sequence of the peptide of the present invention.

Regarding the aforementioned test method, the test for the solubility of the peptide and/or complex in a solvent may be a solubility measurement. For the solubility measurement, the solvent is not limited and may be freely selected according to the purpose. Furthermore, regarding the solubility measurement method, a known method may be appropriately selected according to the type of solvent.
The test for binding ability to VEGFR-2 may be a measurement of the binding ability to VEGFR-2 and is not limited, but may include surface plasmon resonance (SPR) assays, Scatchard analysis, and/or radioimmunoassay (RIA), enzyme immunoassay (EIA), and competitive binding assay methods such as sandwich assays.

Tests for toxicity to cells and/or tissues may be well-known toxicity evaluation tests using cells and/or tissues, and may, for example, be performed in vitro. Cells and tissues may be those typically used for toxicity evaluation tests for pharmaceuticals, and are not limited thereto.

Test methods for toxicity in experimental animals may be well-known toxicity evaluation tests using experimental animals. Regarding experimental animals, there are no particular restrictions as long as they are commonly used, but examples include mice, rats, guinea pigs, gerbils, hamsters, ferrets, rabbits, dogs, cats, pigs, goats, horses, cattle, birds (e.g., chickens, quails), monkeys, and non-human primates (e.g., macaques, marmosets, rhesus monkeys). The toxicity evaluation tests mentioned above are not limited to these examples but may include standard safety tests typically conducted in nonclinical drug studies. Examples include general toxicity studies (single-dose toxicity studies/repeated-dose toxicity studies), genotoxicity studies (Ames test/chromosome aberration test/ in vitro micronucleus test), carcinogenicity studies, reproductive and developmental toxicity studies (ICH-I, II, III), local irritation studies (eye irritation test, skin irritation test, etc.), other toxicity studies (skin sensitization test, phototoxicity test, antigenicity test), and chemical/bioanalytical studies (TK/PK).

### Abbreviations (General)

Å for angstrom (unit);
BSA for bovine serum albumin;
Boc for tert- butoxycarbonyl group;
ClAc for chloroacetyl;
ClAcOSu for (2,5-dioxopyrrolidin-1-yl) 2-chloroacetate;
DCM for dichloromethane or methylene chloride;
DIPCI for N,N'-diisopropylcarbodiimide;
DIPEA or DIEA for N,N-diisopropylethylamine;
DMSO for dimethyl sulfoxide;
DMF for N,N-dimethylformamide;
DODT for 3,6-dioxa-1,8-octane-dithiol;
DMEM for Dulbecco's Modified Eagle's Medium;
EC50 for 50% effective concentration;
FBS for fetal bovine serum;
Fmoc as 9-fluorenylmethyloxycarbonyl;
Fmoc-Lys(Fmoc)-OH for N²,N⁶-bis(((9H-fluoren-9-yl)methoxy)carbonyl)-L-lysine;
g for gram (unit);
HATU for O-(7-azabenzotriazol-1-yl)-N,N,N',N'-tetramethyluronium hexafluorophosphate;
HPLC for high-performance liquid chromatography;
LC-MS or LC/MS for liquid chromatography-mass spectrometer;
M for molar (unit);
MeCN for acetonitrile;
mg for milligram (unit);
min for minute (unit);
mL for milliliter (unit);
mM for millimolar (unit);
mm for millimeter (unit);
Mpe group, namely an O-3-methyl-pent-3-yl group;
NHS for N-hydroxysuccinimide;
nm for nanometer (unit);
µL for microliter (unit);
OSu for succinimide;
Pbf for 2,2,4,6,7-pentamethyldihydrobenzofuran-5-sulfonyl group;
PEG for polyethylene glycol;
rpm for revolutions per minute (unit);
Sub for dibenzosberyl group;
tBu for tert-butyl group;
TEAA for triethylamine acetate;
TFA for trifluoroacetic acid;
TIS for triisopropylsilane; and
Trt or Tr for trityl group.
Abbreviations (Non-natural amino acids)
Ahp for (S)-2-aminoheptanoic acid (CAS No. 44902-02-5);
alT for allo-threonine (CAS No.:28954-12-3);
Atb for (S)-2-amino-4,4-dimethylpentanoic acid (CAS No.: 57224-50-7);
Atp for (2S)-2-amino-3-(oxan-4-yl)propanoic acid (CAS No.: 1344910-91-3);
Bph for (S)-3-[(1,1'-biphenyl)-4-yl]-2-aminopropanoic acid (CAS No.: 155760-02-4);
Cha for (S)-2-amino-3-cyclohexylpropanoic acid (CAS No.: 27527-05-5);
Chg for (S)-2-amino-2-cyclohexylacetic acid (CAS No.: 14328-51-9);
Cit for L-citrulline (CAS No. 372-75-8);
cPEG9c for 4,7,10,13,16,19,22,25,28-nonaoxahentriacontanedioic acid (CAS No.: 1268488-70-5);
cPEG13c for 4,7,10,13,16,19,22,25,28,31,34,37,40-tridecaoxatritetracontanedioic acid (CAS No.:2225903-66-0);
cPEG17c for 4,7,10,13,16,19,22,25,28,31,34,37,40,43,46,49,52-heptadecaoxapentapentacontanedioic acid (CAS No.:2226897-74-9);
F4aao for (S)-2-amino-3-(4-(carboxymethoxy)phenyl)propanoic acid (CAS No.: 24558-63-2);
F4C for (S)-2-amino-3-(4-chlorophenyl)propanoic acid (CAS No.: 14173-39-8);
F4COO for 4-carboxyl-L-phenylalanine (CAS No. 126109-42-0);
F4G for (S)-2-amino-3-(4-guanidinophenyl)propanoic acid (CAS No.: 59574-11-7);
H4Py for (S)-2-amino-4-(pyridin-4-yl)butanoic acid (CAS No.: 1240588-62-8);
Har for N6-carbamoyl-L-lysine (CAS No.: 156-86-5);
Hcit for N6-Carbamoyl-L-lysine (CAS No.: 1190-49-4);
Hgl for L-2-aminoadipic acid (CAS No. 1118-90-7);
Hph for (S)-2-amino-4-phenylbutanoic acid (CAS No.: 943-73-7);
Hty for homo-L-tyrosine (CAS No. 221243-01-2);
Hyp for Hydroxyproline (CAS No.: 51-35-4);
MetO2 for (S)-2-Amino-4-(methylsulfonyl)butanoic acid (CAS No.: 7314-32-1);
NHS-cPEG9c-NHS for Bis(2,5-dioxopyrrolidin-1-yl)4,7,10,13,16,19,22,25,28-nonaoxahexatriacontanediate (CAS No.: 1008402-79-6);
NHS-cPEG13c-NHS for bis(2,5-dioxopyrrolidin-1-yl)4,7,10,13,16,19,22,25,28,31,34,37,40-tridecaoxatritetracontanedioate (Bis-PEG13-NHS ester, CAS No.2221949-00-2);
NHS-cPEG17c-NHS for bis(2,5-dioxopyrrolidin-1-yl)4,7,10,13,16,19,22,25,28,31,34,37,40,43,46,49,52-heptadecaoxapentapentacontane-diolate (Bis-PEG17-NHS ester, CAS No. 2221948-93-0)
NHS-OCOPEG13OCO-NHS for bis(2,5-dioxopyrrolidin-1-yl)(3,6,9,12,15,18,21,24,27,30,33-undecaoxapentatriacontane-1,35-diyl)biscarbonate(Nippon Oil Corporation);
NHS-OCOPEG17OCO-NHS for bis(2,5-dioxopyrroldin-1-yl)(3,6,9,12,15,18,21,24,27,30,33,36,39,42,45- Pentadecahydro-heptatetracontane-1,47-diylz)-bis carbonate (Nippon Oil Corporation);
OCOPEG17OCO for 3,6,9,12,15,18,21,24,27,30,33,36,39,42,45-Pentadecahydro-heptatetracontane-1,47-diyl bis(hydrogen carbonate) (Nippon Oil Corporation);
PEG8c for 1-amino-3,6,9,12,15,18,21,24-octaoxaheptacosan-27-oic acid (CAS NO.: 756526-04-2);
PEG12c for 1-amino-3,6,9,12,15,18,21,24,27,30,33,36-dodecaoxanonatriacontan-39-oic acid (CAS NO.: 1415408-69-3);
3Py6NH2 for (S)-2-amino-3-(6-aminopyridin-3-yl)propanoic acid (CAS No.: 1269968-61-7);
4Py2NH2 for (S)-2-amino-3-(2-aminopyridin-4-yl)propanoic acid (CAS No.: 1269969-46-1);
Tbg for (S)-2-Amino-3,3-dimethylbutanoic acid (CAS No.: 20859-02-3);
W7N for (S)-2-Amino-3-(1H-pyrrolo[2,3-b]pyridin-3-yl)propanoic acid (CAS No.: 49758-35-2);
Yph for (S)-2-Amino-3-(4-phenoxyphenyl)propanoic acid (CAS No.: 150351-64-7).

### EXAMPLES

The present invention is described in more detail below with reference to the Examples. However, the present invention is not limited to the following Examples. A person skilled in the art may easily modify or change the present invention based on the description of the present specification, and these modifications and changes are included in the technical scope of the present invention.

### Chemical synthesis

All raw materials, building blocks, reagents, acids, bases, solid-phase resins, and solvents used in the chemical syntheses of the following Examples were used as they were commercially available, or they can be synthesized by those skilled in the art using organic chemical techniques. An amino acid comprising a protecting group was used as it was commercially available unless otherwise noted.

The elongation of a peptide chain in the solid-phase resin was performed by using the resins described in the Examples as starting materials and using commonly used peptide coupling reaction conditions and Fmoc removal reaction conditions. The reactions were performed using Syro I or Syro II available from Biotage, or Liberty Blue, Liberty Blue HT12, or Liberty Prime available from CEM, which is an automated peptide synthesizer, according to the manufacturer's manual.

The resin used was NovaPEG Rink Amide resin or Seiber Amide resin, and the amount used ranged from 5 mg to 2 g for each peptide.

The amount of the reagent cocktail used for the deprotection of the side chain and the cleavage of the side chain from the solid-phase resin is 4 mL to 50 mL for each peptide, and solutions of the following compositions were used.
A: TFA/H₂O/TIS/DODT (92.5/2.5/2.5/2.5)
B: TFA/H₂O/TIS/DODT (90/2.5/2.5/5)

Common amino acids that were used are listed below, and the side chain protecting groups are indicated in parentheses:

**[Table 1-1]**

| Fmoc Amino Acid | CAS No. |
|---|---|
| Fmoc-3Py6NH2(Boc)-OH | 2699074-67-2 |
| Fmoc-4Py2NH2(Boc)-OH | 2640471-43-6 |
| Fmoc-Ahp-OH | 1197020-22-6 |
| Fmoc-Ala-OH | 35661-39-3 |
| Fmoc-alT(tBu)-OH | 201481-37-0 |
| Fmoc-Arg(Pbf)-OH | 154445-77-9 |
| Fmoc-Asn(Trt)-OH | 132388-59-1 |
| Fmoc-Asp(Mpe)-OH | 180675-08-5 |
| Fmoc-Atb-OH | 139551-74-9 |
| Fmoc-Atp-OH | 368866-34-6 |
| Fmoc-Bph-OH | 199110-64-0 |
| Fmoc-Cha-OH | 135673-97-1 |
| Fmoc-Chg-OH | 161321-36-4 |
| Fmoc-Cit-OH | 133174-15-9 |
| Fmoc-Cys(Trt)-OH | 103213-32-7 |
| Fmoc-F4aao(tBu)-OH | 181951-92-8 |
| Fmoc-F4C-OH | 175453-08-4 |
| Fmoc-F4COO(tBu)-OH | 183070-44-2 |
| Fmoc-F4G(Pbf,Sub)-OH | 2803482-69-9 |
| Fmoc-Gln(Trt)-OH | 132327-80-1 |
| Fmoc-Glu(tBu)-OH | 71989-18-9 |
| Fmoc-Gly-OH | 29022-11-5 |
| Fmoc-H4Py-OH | 273222-04-1 |
| Fmoc-Har(Pbf)-OH | 1159680-21-3 |

**[Table 1-2]**

| Fmoc Amino Acid | CAS No. |
|---|---|
| Fmoc-Hcit-OH | 201485-17-8 |
| Fmoc-Hph-OH | 132684-59-4 |
| Fmoc-Hty(tBu)-OH | 204384-69-0 |
| Fmoc-Hyp(tBu)-OH | 122996-47-8 |
| Fmoc-Ile-OH | 71989-23-6 |
| Fmoc-Leu-OH | 35661-60-0 |
| Fmoc-Lys(Boc)-OH | 71989-26-9 |
| Fmoc-Lys(Fmoc)-OH | 78081-87-5 |
| Fmoc-MetO2-OH | 163437-14-7 |
| Fmoc-PEG 12c-OH | 1952360-91-6 |
| Fmoc-PEG8c-OH | 756526-02-0 |
| Fmoc-Phe-OH | 35661-40-6 |
| Fmoc-Ser(Trt)-OH | 111061-56-4 |
| Fmoc-Tbg-OH | 132684-60-7 |
| Fmoc-Thr(Trt)-OH | 133180-01-5 |
| Fmoc-Trp(Boc)-OH | 143824-78-6 |
| Fmoc-Tyr(tBu)-OH | 71989-38-3 |
| Fmoc-Val-OH | 68858-20-8 |
| Fmoc-W7N-OH | 737007-45-3 |
| Fmoc-Yph-OH | 180414-93-1 |

Unless otherwise specified, the crude purified peptides obtained were purified using one of the following reverse-phase fractionation purification systems: A), B), C), or D).
A): Shimadzu prep-HPLC system(LC-20AP,SPD-M20A,CTO-20AC,and CBM-20A);
B): Waters AutoPurification System;
C): Waters AutoPurification System with SQD;
D): Waters Preparative HPLC System.

Unless otherwise specified, the columns used were one of the following: a) to n).
a) Jeanious One-Column 20mmI.D.x150mmL
b) Kinetex EVO C18 5µm 21.2x150mm
c) Waters XBridge BEH Prep OBD Amide 5µm 19x150mm
d) Waters XBridge C18 19x150mm
e) Waters XBridge C18 5µm 19x150mm
f) Waters XBridge C18 5µm 30x150mm
g) Waters XBridge C18 5µm 50x150mm
h) Waters XSelect C18 19x150mm
i) Waters XSelect C18 30x150mm
j) Waters XSelect C18 50x150mm
k) Waters XSelect C18 5µm 19x150mm
l) Waters XSelect C18 5µm 30x150mm
m) Waters XSelect CSH Prep C18 5µm OBD 30x150mm
n) Waters XSelect CSH Prep C18 5µm OBD 50x250mm

The structure of the chemically synthesized peptides was determined by ESI-MS(+) in mass spectrometry, where the molecular weight was calculated by considering the amino acids used according to the target sequence and the building blocks used as needed. The term "ESI-MS(+)" refers to electrospray ionization mass spectrometry performed in positive ion mode. The detected masses are reported in "m/z" units. Compounds with molecular weights roughly greater than 1,000 were detected as multivalent ions at a high frequency.

### Basic Analytical Instruments and Basic Conditions

For the mass spectrometry of the peptides synthesized in the following Examples, the following basic analyzers and basic conditions 1 or 2 were used unless otherwise noted. The slope B (%) was analyzed using any of the x/y conditions.
Instrument: Shimadzu LC/MS system (LC-20ADXR, CTO-20AC, SPD-M20A, SIL-20AXR, CBM-20A, and LCMS-2020);
Column: Kinetex EVO C18, 2.6 µm, 2.1 x 150 mm, 100 Å;
Column temperature: 60°C;
Mobile phase A: 0.025% TFA in H₂O;
Mobile phase B: 0.025% TFA in MeCN;
Flow rate: 0.5 mL/min;
Wavelength: 225 nm PDA;
Slope B (%):
   x: 20% to 60%/7.15 min, 60% to 95%/0.3 min, 95% to 95%/1.55 min;
   y: 40% to 80%/7.15 min, 80% to 95%/0.3 min, 95% to 95%/1.55 min.

### Example 1: Synthesis of Peptide Complex (Dimer Structure No. 52)

In this example, a peptide complex having the following structure was synthesized (a complex of Peptide Sequence No. 47 and Linker Structure No. 6, corresponding to Dimer Structure No. 52 in Table 4).

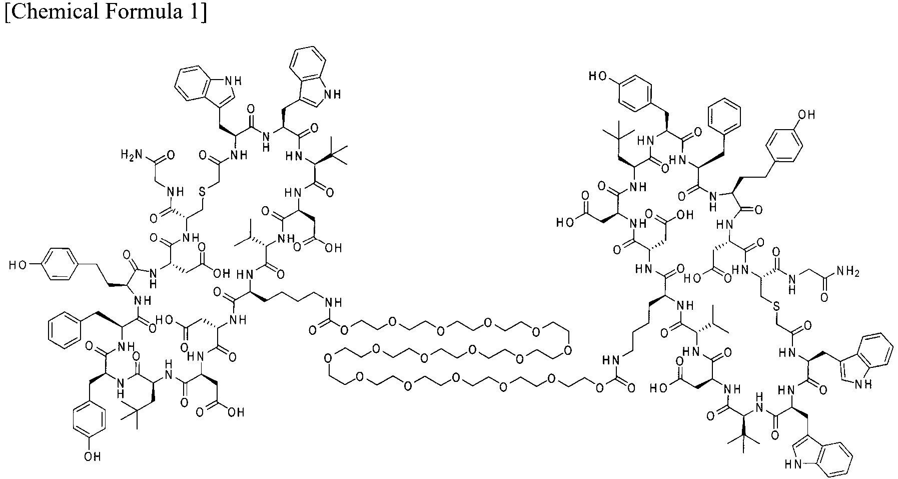

Using Sieber amide resin (Watanabe Chemical, 0.60 mmol/g), the target peptide was synthesized starting with Fmoc group removal according to the general method described above. For this, the Liberty Blue HT12 from CEM was used as the solid-phase synthesizer, and synthesis was performed according to the manufacturer's manual. For each residue introduction, Fmoc-AA/DIPCI/Oxymapure (4.2 equivalents /8 equivalents /4 equivalents) was used per resin equivalent. Reactions were performed once at 90°C for 3 minutes in DMF. However, the 3rd and 9th residues were reacted once at 90°C for 10 minutes. The 14th residue was reacted once at 50°C for 15 minutes.

Fmoc removal was performed using either a 10% pyrrolidine DMF solution at 90°C for 1 minute or at 50°C for 90 seconds.

Chloroacetyl group introduction was performed by adding a DMF solution of ClAcOSu (5 equivalents) to the solid-phase resin and shaking at room temperature for 60 minutes.

Side-chain deprotection and removal from the solid-phase resin were performed by first washing the resin obtained after the chloroacetyl group introduction step with DMF, followed by methylene chloride and diethyl ether. The resin was then dried under reduced pressure. Reaction cocktail A (a mixture of TFA/H₂O/ TIS/DODT in a volume ratio of 92.5:2.5:2.5:2.5) was added to the reaction vessel containing the solid-phase resin and shaked at room temperature for 60 minutes. The reaction mixture was filtered and recovered through frit. Adding this filtrate to a chilled excess of diethyl ether/hexane (1/1) mixed solvent produced a white turbid precipitate. This mixture was centrifuged, and the supernatant was decanted. The resulting solid was washed again with chilled diethyl ether and dried under reduced pressure. This solid was used in the subsequent cyclization reaction.

For the peptide cyclization reaction, the peptide was dissolved in DMSO/H₂O (9/1) to achieve a final concentration of 4.2 mM based on the molar amount of the solid-phase resin. Triethylamine (20 equivalents) was added, and the mixture was shaken at room temperature for 1 hour before adding acetic acid. The resulting reaction solution was concentrated under reduced pressure using a Genevac HT-12. The crude product obtained was purified under the following conditions (Column: Waters XSelect C18 5µm 50x250mm;
Mobile phase: A = 20 mM TEAA- in H₂O, B = 20 mM TEAA in MeCN, C = 0.2 M TEAA in H₂O, D = MeCN
Temperature: 50°C;
Gradient (%A conc): 0.1% over 5.0 min, then 0.1% over 0.1 min to 0.1-100%, thereafter 5.1 min (100%-%B); (%B conc): 0% over 5.1 min, then 0-4.2% over 1.9 min, then 4.2-20.5% over 3 min, then 20.5-25.6% over 15.5 min, then 25.6-60% over 1.5 min, then 60% over 4 min; (%C conc): 99.9% over 5.0 min, then 99.9-0% over 0.1 min, 0% after 5.1 min; (%D conc): 0%
Flow rate: 18 mL/min for 5.1 min, then (18 mL/min-118 mL/min) for 1.9 min, then 118 mL/min)
After freeze-drying, 0.1% TFA in H₂O-MeCN (1:1, 100 mL) was added, freeze-dried again, and the resulting Peptide A was used for peptide complex synthesis.

For peptide complex synthesis, peptide A was dissolved in DMSO to a final concentration of 25 mM. NHS-OCOPEG17OCO-NHS (0.4 equivalents) and DIEA (10 equivalents) were added, followed by shaking at room temperature for 1 hour. Acetic acid was then added.

The crude product was purified under the following conditions (
Column: Waters XBridge C18, 19 x 150 mm;
Mobile phase: A = 0.1% TFA in H₂O, B = 0.1% TFA in MeCN;
Temperature: 50°C;
Gradient (%B conc): 15-40% over 3 min, then 40-45% over 8 min, then 45-60% over 1 min; Flow rate: 17 mL/min.

The purity of the target compound was calculated from the area ratio of the LC/MS (UV wavelength 225 nm) chromatogram under the following analytical conditions and was 94.86%.

### Analysis conditions:

Retention time = 5.7 min;
Column: Kinetix EVO C18 2.6 µm 2.1 x 150 mm, 100 Å;
Mobile phase:
   A = 0.025% TFA in H₂O, B = 0.025% TFA in MeCN;
   Temperature: 60°C;
   Gradient (%B conc): 20-60% over 7.15 min, then 60-95% over 0.30 min, then 95-95% over 1.55 min;
   Flow rate: 0.5 mL/min;
   ESI-MS(+) observed m/z = 1197 (M+4H)4+.)

### Example 2: Synthesis of Peptide Complex (Dimer Structure No. 1)

In this example, a peptide complex having the following structure was synthesized (a complex of Peptide Sequence No. 3 and Linker Structure No. 1, corresponding to Dimer Structure No. 1 in Table 4).

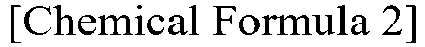

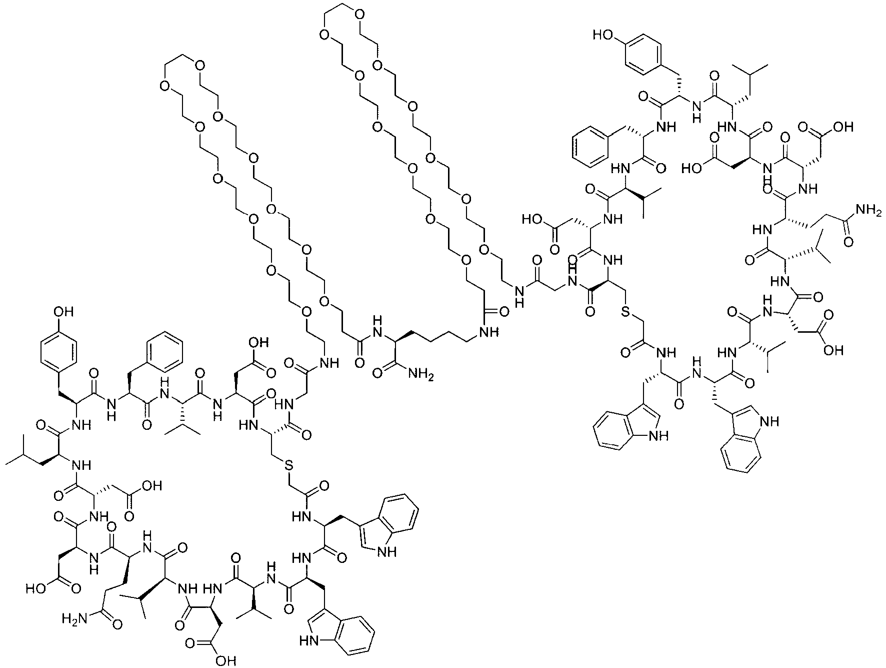

Using Sieber amide resin (Watanabe Chemical, 0.65 mmol/g), synthesis commenced by removing Fmoc groups via the general method described above. Following introduction of Fmoc-Lys (Fmoc)-OH, followed by introduction of the linker segment PEG and individual Fmoc amino acids to synthesize the target peptide. For this, Syro I from Biotage was used as the solid-phase synthesizer, and synthesis was performed according to the manufacturer's manual. For each residue introduction, Fmoc-AA/HATU/DIEA (9.45 equivalents/9 equivalents/18.8 equivalents) was used per resin equivalent. Reactions were conducted in DMF at 75°C for 20 minutes, repeated twice. However, the 2nd residue was reacted three times at 75°C for 20 minutes each. The 4th residue was reacted twice at room temperature for 60 minutes each. The 14th residue was reacted twice at room temperature for 30 minutes each. The 15th residue, the linker portion Fmoc-PEG12c, and Fmoc-Lys(Fmoc)-OH were introduced by reacting twice at 75°C for 20 minutes each.

Fmoc removal was performed by reacting with a 20% piperidine solution in DMF at room temperature for 5 minutes. After removing the solution, the sample was reacted again with a 20% piperidine solution in DMF at room temperature for 15 minutes.

Chloroacetylation was performed by removing the Fmoc protection from the α-amino group of the Fmoc-protected peptide retained on the solid-phase resin using the method described above. This was followed by adding chloroacetic acid (0.5 M DMF solution, 10 equivalents), HCTU (0.49 M DMF solution, 9.8 equivalents), and DIEA (0.5 M DMF solution, 10 equivalents) to the solid-phase resin and shaking at room temperature for 30 minutes.

Side-chain deprotection and cleavage from the solid-phase resin were performed by adding reaction cocktail A (a mixture of TFA/H₂O/TIS/DODT in a volume ratio of 92.5:2.5:2.5:2.5) to the reaction vessel containing the solid-phase resin, followed by vigorous shaking and shaking at room temperature for 60 minutes. The reaction mixture was filtered and recovered from the frit. Adding this filtrate to a chilled excess of diisopropyl ether/hexane (1/1) mixed solvent produced a white turbid precipitate. This mixture was centrifuged, and the supernatant was decanted. The resulting solid was washed with chilled diisopropyl ether/hexane (1/1) mixed solvent and dried under reduced pressure for 60 minutes. The solid obtained was used in the subsequent cyclization reaction.

For the peptide cyclization reaction, the peptide was dissolved in DMSO/MeCN/H₂O (18/1/1) to achieve a final concentration of 1 mM based on the molar amount of the solid-phase resin. Triethylamine (20 equivalents) was added, and the mixture was stirred at room temperature for 3 hours. The resulting reaction solution was concentrated under reduced pressure using a Genevac EZ-2 Elite.

The crude product was purified under the following conditions: (
Column: Waters XBridge(Registered Trademark) C18, 50 x 150 mm;
Mobile phase: A = 0.1% TFA in H₂O, B = 0.1% TFA in MeCN;
Temperature: 40°C;
Gradient (%B conc): 9-34% over 3 min, then 34-39% over 8 min, then 39-60% over 1 min;
Flow rate: 120 mL/min.

The purity of the target compound was calculated from the area ratio of the LC/MS (UV wavelength 225 nm) chromatogram under the following analytical conditions and was 86.83%.

### Analysis conditions:

Retention time = 5.3 min;
Column: Kinetix EVO C18, 2.6 µm, 2.1 x 150 mm, 100 Å;
Mobile phase: A = 0.025% TFA in H₂O, B = 0.025% TFA in MeCN;
Temperature: 60°C;
Gradient (%B conc): 20-60% over 7.15 min, then 60-95% over 0.30 min, followed by 95-95% over 1.55 min;
Flow rate: 0.5 mL/min;
ESI-MS(+) observed m/z = 1023 (M+5H)5+.)

### Example 3: Synthesis of Peptide Complex (Dimer Structure No. 43)

In this example, a peptide complex having the following structure was synthesized (a complex of Peptide Sequence No. 42 and Linker Structure No. 4, corresponding to Dimer Structure No. 43 in Table 4).

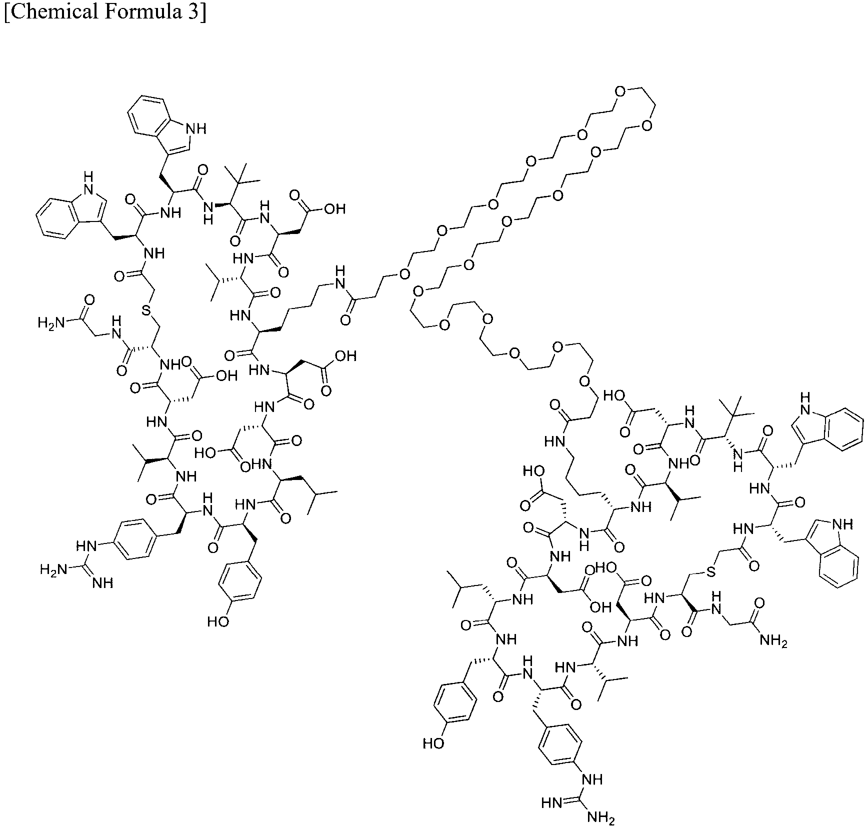

Using Sieber amide resin (Watanabe Chemical, 0.57 mmol/g), the target peptide was synthesized starting with Fmoc group removal according to the general method described above. For this, Biotage's Syro I was used as the solid-phase synthesizer, and synthesis was performed according to the manufacturer's manual. For each residue introduction, Fmoc-AA/HATU/DIPEA (4.2 equivalents/4 equivalents/8.4 equivalents) was used per resin equivalent. Reactions were performed in DMF at 75°C for 20 minutes, repeated twice. However, for the 13th residue, the reaction was performed at 75°C for 30 minutes, repeated twice. The 14th residue was reacted once at room temperature for 30 minutes. The 15th residue was reacted once at 75°C for 20 minutes.

Fmoc removal was performed by reacting with a 20% piperidine solution in DMF at room temperature for 5 minutes. After removing the solution, the sample was reacted again with the same 20% piperidine solution in DMF at room temperature for 15 minutes.

The introduction of the chloroacetyl group was performed by first removing the Fmoc protection from the α-amino group of the Fmoc-protected peptide obtained in the previous step using the aforementioned method. This was followed by adding a DCM/DMF solution of ClAcOSu, which was prepared from DIPCI (10 equivalents), HOSu (10 equivalents), and chloroacetic acid (10 equivalents) in DCM then adding an equal volume of DMF. This mixture was shaken at room temperature for 60 minutes.

For side-chain deprotection and isolation from the solid-phase resin, the reaction vessel containing the solid-phase resin was treated with reaction cocktail A (a mixture of TFA/H₂O/TIS/DODT in a volume ratio of 92.5:2.5:2.5:2.5) and shaken at room temperature for 60 minutes. The reaction mixture was filtered and recovered from the frit. Adding this filtrate to excess chilled diisopropyl ether produced a white precipitate. This mixture was centrifuged, and the supernatant was decanted. The resulting solid was washed with chilled diethyl ether and dried under reduced pressure. This solid was used in the subsequent cyclization reaction.

For the peptide cyclization reaction, the peptide was dissolved in DMSO/H₂O (9/1) to achieve a final concentration of 5 mM based on the molar amount of the solid-phase resin. Triethylamine (10 equivalents) was added, and the mixture was stirred at room temperature for 15 hours. The reaction solution was concentrated under reduced pressure using a Genevac EZ-2 Elite, yielding the unpurified cyclic peptide B.

For peptide complex synthesis, the above-obtained peptide B was dissolved in DMSO to a concentration of 25 mM. Ten equivalents of diisopropylethylamine were added, followed by 0.4 equivalents of NHS-cPEG17c-NHS. The mixture was shaken at room temperature for 30 minutes, then acetic acid was added.

The crude product was purified under the following conditions: (
Column: Kinetix EVO C18 5µm 21.2x150mm;
Mobile phase: A = 0.1% TFA in H₂O, B = 0.1% TFA in MeCN;
Temperature: 60°C;
Gradient (%B conc): 5-30% over 3 min, then 30-35% over 8 min, then 35-60% over 1 min;
Flow rate: 20 mL/min.

The purity of the target compound was calculated from the area ratio of the LC/MS (UV wavelength 225 nm) chromatogram under the following analytical conditions and was 87.55%.

### Analytical conditions:

Retention time = 4.5 min;
Column: Kinetix EVO C18, 2.6 µm, 2.1 x 150 mm, 100 Å;
Mobile phase: A = 0.025% TFA in H₂O, B = 0.025% TFA in MeCN;
Temperature: 60°C;
Gradient (%B conc): 20-60% over 7.15 min, then 60-95% over 0.30 min, then 95-95% over 1.55 min; Flow rate: 0.5 mL/min;
ESI-MS(+) observed m/z = 1194 (M+4H)4+).

### Example 4: Synthesis of Various Peptides

In this example, various peptide complexes were chemically synthesized in the same manner as in Examples 1-3. The sequences of the synthesized cyclic peptides are shown in Table 2, the linker structures are shown in Table 3, and the peptide complexes formed by dimerization of the cyclic peptides via the linker are shown in Table 4. The Conjugation No. in Table 4 indicates the position of the amino acid to which the linker is attached within the amino acid sequence designated by each peptide sequence number.

For peptide sequence numbers 14-35 and 37-51 in Tables 2 and 4, it is preferable that the carboxyl group at the C-terminus of the 15th Gly be converted to an amide.

The synthesized peptide complexes were analyzed under any of the analytical conditions described in the basic analytical apparatus and basic conditions above, and their structures were confirmed by ESI-MS(+) in mass spectrometry. Table 4 shows the obtained ESI-MS(+) observations, retention times, valences, and the concentration gradient (%) of mobile phase B used for analysis.

**Table 2. Sequence of the synthesized cyclic peptide**

| [Table 2-1] | | | | | | | | | | | | | | | |
|---|---|---|---|---|---|---|---|---|---|---|---|---|---|---|---|
| **Seq** | **Peptide SEQ** | | | | | | | | | | | | | | |
| | **1** | **2** | **3** | **4** | **5** | **6** | **7** | **8** | **9** | **10** | **11** | **12** | **13** | **14** | **15** |
| 3 | W | W | V | D | V | Q | D | D | L | Y | F | V | D | C | G |
| 4 | W | W | V | D | V | Ahp | D | D | L | Y | F | V | D | C | G |
| 5 | W | W | V | D | V | W | D | D | L | Y | F | V | D | C | G |
| 6 | W | W | V | D | V | Hph | D | D | L | Y | F | V | D | C | G |
| 7 | W | W | V | D | V | Cha | D | D | L | Y | F | V | D | C | G |
| 8 | W | W | V | D | V | F4COO | D | D | L | Y | F | V | D | C | G |
| 9 | W | W | V | D | V | Q | G | D | L | Y | F | V | D | C | G |
| 10 | W | W | V | D | V | Q | D | D | L | Y | Bph | V | D | C | G |
| 11 | W | W | V | D | V | Q | D | D | L | Y | F | Hph | D | C | G |
| 12 | W | W | V | D | V | Q | D | D | L | Y | F | V | A | C | G |
| 13 | W | W | V | D | V | Q | D | D | L | Y | F | V | S | C | G |
| 14 | W | W | V | D | V | K | D | D | L | Y | F | V | D | C | G |
| 15 | W | W | V | D | Tbg | K | D | D | L | Y | F | V | D | C | G |
| 16 | W | W | V | D | V | K | D | D | L | Y | Yph | V | D | C | G |
| 17 | W | W | V | D | V | K | D | D | L | Y | F4G | V | D | C | G |
| 18 | W | W | V | D | V | K | D | D | L | Y | F | Hty | D | C | G |
| 19 | W | W | Tbg | D | V | K | D | D | L | Y | Yph | V | D | C | G |
| 20 | W | W | Tbg | D | V | K | D | D | L | Y | Yph | Hty | D | C | G |
| 21 | Hcit | W | Tbg | D | V | K | D | D | L | Y | F | Hty | D | C | G |
| 22 | 4Py2NH2 | W | Tbg | D | V | K | D | D | L | Y | F | Hty | D | C | G |
| 23 | 3Py6NH2 | W | Tbg | D | V | K | D | D | L | Y | F | Hty | D | C | G |
| 24 | F4aao | W | Tbg | D | V | K | D | D | L | Y | F | Hty | D | C | G |
| 25 | W | W | Chg | D | V | K | D | D | L | Y | F | Hty | D | C | G |
| 26 | W | W | Tbg | Q | V | K | D | D | L | Y | F | Hty | D | C | G |

**[Table 2-2]**

| | | | | | | | | | | | | | | | |
|---|---|---|---|---|---|---|---|---|---|---|---|---|---|---|---|
| 27 | W | W | Tbg | D | aIT | K | D | D | L | Y | F | Hty | D | C | G |
| 28 | W | W | Tbg | D | Chg | K | D | D | L | Y | F | Hty | D | C | G |
| 29 | W | W | Tbg | D | v | K | D | D | N | Y | F | Hty | D | C | G |
| 30 | W | W | Tbg | D | V | K | D | D | L | Y | F4C | Hty | D | C | G |
| 31 | W | W | Tbg | D | V | K | D | D | L | Y | F | Chq | D | C | G |
| 32 | W | W | Tbg | D | V | K | D | D | L | Y | F | H4Py | D | C | G |
| 33 | W | W | Tbg | D | V | K | D | D | L | Y | F | Hty | S | C | G |
| 34 | W | W | Tbg | D | V | K | D | D | L | Y | F | Hty | F | C | G |
| 35 | W | W | Tbg | D | V | K | D | D | L | Y | F | Hty | F4aao | C | G |
| 36 | W | W | Tbg | D | V | Ahp | D | D | L | Y | F | Hty | D | C | G |
| 37 | W | W | V | D | V | K | G | V | N | Y | F | Hty | D | C | G |
| 38 | W | W | V | MetO2 | V | K | D | D | L | Y | F | Hty | D | C | G |
| 39 | W | W | V | D | V | K | D | D | L | Y | Yph | V | Har | C | G |
| 40 | W | W | Hty | D | V | K | D | D | L | Y | F4G | V | D | C | G |
| 41 | W | W | V | D | V | K | D | D | L | Y | F4G | Hty | Hyp | C | G |
| 42 | W | W | Tbg | D | V | K | D | D | L | Y | F4G | V | D | C | G |
| 43 | W | W | Tbg | D | V | K | D | D | L | Y | F | Hty | D | C | G |
| 44 | W | W | Tbg | D | V | K | D | D | L | Y | F4G | Hty | D | C | G |
| 45 | W7N | W | Tbg | D | V | K | D | D | L | Y | F | Hty | D | C | G |
| 46 | W | W | Tbg | D | V | K | N | D | L | Y | F | Hty | D | C | G |
| 47 | W | W | Tbg | D | V | K | D | D | Atb | Y | F | Hty | D | C | G |
| 48 | W | W | Tbg | D | V | K | D | D | Atb | Y | F4G | Hty | D | C | G |
| 49 | W | W | Tbg | D | V | K | D | D | Atb | Y | F4G | Hty | Hyp | C | G |
| 50 | W | W | Hty | D | V | K | D | D | Atb | Y | F4G | V | D | C | G |
| 51 | W | W | Hty | D | V | K | D | D | Atb | Y | F4G | Hty | D | C | G |

**Table 3: Structure of Linkers**

| [Table 3] | | | |
|---|---|---|---|
| Linker Structure No. | Liner Sequence | | |
| 1 | PEG12c | K | PEG12c |
| 2 | cPEG9c | | |
| 3 | cPEG13c | | |
| 4 | cPEG17c | | |
| 5 | OCOPEG13OCO | | |
| 6 | OCOPEG17OCO | | |
| 7 | K | cPEG17c | K |

**Table 4: Peptide Complex**

| **[Table 4-1]** | | | | | | | |
|---|---|---|---|---|---|---|---|
| **Dimer Structure No.** | **Seq** | **Linker Structure No.** | **Conjugation No.** | **Ret time** | **ESI (m/z)** | **[M+XH]X+** | **QC condition** |
| | | | | | | | **Concentration gradient of B** |
| 1 | 3 | 1 | 15 | 5.3 | 1023 | 5 | x |
| 2 | 4 | 1 | 15 | 3.4 | 1278 | 4 | y |
| 3 | 5 | 1 | 15 | 3.0 | 1307 | 4 | y |
| 4 | 6 | 1 | 15 | 3.4 | 1295 | 4 | y |
| 5 | 7 | 1 | 15 | 3.8 | 1033 | 5 | y |
| 6 | 8 | 1 | 15 | 5.7 | 1310 | 4 | x |
| 7 | 9 | 1 | 15 | 5.0 | 1249 | 4 | x |
| 8 | 10 | 1 | 15 | 6.2 | 1316 | 4 | x |
| 9 | 11 | 1 | 15 | 6.0 | 1309 | 4 | x |
| 10 | 12 | 1 | 15 | 5.4 | 1005 | 5 | x |
| 11 | 13 | 1 | 15 | 5.3 | 1011 | 5 | x |
| 12 | 3 | 7 | 15 | 5.1 | 1222 | 4 | x |
| 13 | 14 | 2 | 6 | 5.5 | 1427 | 3 | x |
| 14 | 14 | 3 | 6 | 5.6 | 1485 | 3 | x |
| 15 | 15 | 4 | 6 | 5.4 | 1166 | 4 | x |
| 16 | 16 | 4 | 6 | 6.4 | 1205 | 4 | x |
| 17 | 17 | 4 | 6 | 4.4 | 1187 | 4 | x |
| 18 | 18 | 4 | 6 | 5.5 | 1198 | 4 | x |
| 19 | 19 | 4 | 6 | 6.3 | 1212 | 4 | x |
| 20 | 20 | 4 | 6 | 6.5 | 1250 | 4 | x |
| 21 | 21 | 4 | 6 | 4.6 | 1197 | 4 | x |
| 22 | 22 | 4 | 6 | 3.9 | 1193 | 4 | x |
| 23 | 23 | 4 | 6 | 3.9 | 1193 | 4 | x |
| 24 | 24 | 4 | 6 | 4.9 | 1222 | 4 | x |

**[Table 4-2]**

| | | | | | | | |
|---|---|---|---|---|---|---|---|
| 25 | 25 | 4 | 6 | 6.0 | 974 | 5 | x |
| 26 | 26 | 4 | 6 | 5.2 | 1211 | 4 | x |
| 27 | 27 | 4 | 6 | 5.4 | 1205 | 4 | x |
| 28 | 28 | 4 | 6 | 5.8 | 1224 | 4 | x |
| 29 | 29 | 4 | 6 | 5.5 | 1205 | 4 | x |
| 30 | 30 | 4 | 6 | 5.9 | 1221 | 4 | x |
| 31 | 31 | 4 | 6 | 5.8 | 1186 | 4 | x |
| 32 | 32 | 4 | 6 | 4.6 | 1197 | 4 | x |
| 33 | 33 | 4 | 6 | 5.5 | 1190 | 4 | x |
| 34 | 34 | 4 | 6 | 6.5 | 1220 | 4 | x |
| 35 | 35 | 4 | 6 | 5.8 | 1257 | 4 | x |
| 36 | 36 | 1 | 15 | 3.3 | 1324 | 4 | y |
| 37 | 37 | 5 | 6 | 5.2 | 1470 | 3 | x |
| 38 | 38 | 5 | 6 | 5.3 | 1163 | 4 | x |
| 39 | 39 | 5 | 6 | 5.0 | 1174 | 4 | x |
| 40 | 40 | 5 | 6 | 43 | 1168 | 4 | x |
| 41 | 41 | 5 | 6 | 3.9 | 1167 | 4 | x |
| 42 | 14 | 4 | 6 | 5.4 | 1166 | 4 | x |
| 43 | 42 | 4 | 6 | 4.5 | 1194 | 4 | x |
| 44 | 43 | 4 | 6 | 5.5 | 1204 | 4 | x |
| 45 | 44 | 4 | 6 | 4.2 | 1233 | 4 | x |
| 46 | 45 | 4 | 6 | 4.2 | 1205 | 4 | x |
| 47 | 46 | 4 | 6 | 5.1 | 1204 | 4 | x |
| 48 | 47 | 4 | 6 | 5.5 | 1211 | 4 | x |
| 49 | 47 | 5 | 6 | 5.2 | 1154 | 4 | x |
| 50 | 14 | 6 | 6 | 5.9 | 1144 | 4 | x |
| 51 | 42 | 6 | 6 | 4.8 | 1180 | 4 | x |
| 52 | 47 | 6 | 6 | 5.7 | 1197 | 4 | x |
| 53 | 48 | 6 | 6 | 4.1 | 1226 | 4 | x |
| 54 | 49 | 5 | 6 | 4.0 | 1181 | 4 | x |
| 55 | 48 | 5 | 6 | 4.2 | 1182 | 4 | x |
| 56 | 50 | 5 | 6 | 4.3 | 1175 | 4 | x |
| 57 | 51 | 5 | 6 | 4.2 | 1214 | 4 | x |

### Example 5: Evaluation of VEGFR-2 Agonist Activity Using the pVEGFR Alpha LISA Assay

To evaluate the VEGFR-2 activation ability of the VEGFR-2 agonist peptides of the present invention, VEGFR-2 phosphorylation was verified. HUVECs were cultured using EGM^{™} -2 Endothelial Cell Growth Medium-2 BulletKit (LONZA; CC-3162). After detaching cells using Tryple (Thermo Fisher Scientific), they were seeded into a 96-well PDL-coated plate (Corning: 354461) at a density of 24,000 cells per well and cultured for 2 days. For starvation, the medium was replaced with EBM-2 medium (LONZA) containing 0.5% FBS (Thermo Fisher Scientific), 0.1% heparin (included with the above LONZA CC-3162), and 0.1% gentamicin (Nacalai), and the cells were cultured for 18 hours. Subsequently, Recombinant Human VEGF165a Protein (R&D Systems) or the peptide was added, and the cells were cultured in a CO₂ incubator at 37°C. After 10 minutes of stimulation, cells were lysed using the Lysis Buffer included in the AlphaLISA SureFire Ultra VEGFR-2 (p-Tyr1175) Assay Kit (PerkinElmer) to lyse the cells. The procedure was performed according to the kit protocol, and signal detection was performed using a SpectraMax Paradigm multimode microplate reader (Molecular Devices). The obtained signals were analyzed using GraphPad Prism, and % activity was calculated with the maximum signal induced by VEGF165a set as 100% and the unstimulated control as 0%.

The VEGF165a concentration was evaluated at five points (0.08, 0.31, 1.25, 5, 20 nM) using 1/4-fold dilutions starting from 20 nM to determine the maximum value (100%). Peptide concentrations tested were: 3.2, 16, 80 nM for dimer structure number 1; 10, 100 nM for dimer structures numbers 2-41; and 1.25, 5, 20 nM for dimer structures numbers 42-57.

For % activity, peptides showing ≥50% activity at 3.2 nM were designated 1-A, those showing ≥50% activity at 16 nM were designated 1-B, those showing ≥50% activity at 80 nM were designated 1-C, and those showing ≥1% but <50% activity at 80 nM were designated 1-D.

Peptides showing 50% or more activity at 10 nM were designated 2-A, those showing 50% or more activity at 100 nM were designated 2-B, and those showing 1% or more but less than 50% activity at 100 nM were designated 2-C.

Peptides showing 50% or more activity at 1.25 nM were designated 3-A, those showing 50% or more activity at 5 nM were designated 3-B, those showing 50% or more activity at 20 nM were designated 3-C, and those showing 1% or more but less than 50% activity at 20 nM were designated 3-D.

The results are shown in Table 5. Note that none of the compounds corresponded to categories 1-A to 1-C or 3-A in the above classification. As shown in Table 5, the synthesized peptide complexes were demonstrated to possess VEGFR-2 agonist activity that induces VEGFR-2 phosphorylation.

**Table 5. Evaluation of VEGRF-2 Agonist Activity**

| [Table 5-1] | | |
|---|---|---|
| Dimer Structure No. | pVEGFR2 Assay Results | pERK Assay Results |
| 1 | 1-D | 1-D |
| 2 | 2-C | 2-B |
| 3 | 2-C | 2-A |
| 4 | 2-C | 2-A |
| 5 | 2-C | 2-A |
| 6 | 2-C | 2-B |
| 7 | 2-C | 2-B |
| 8 | 2-C | 2-C |
| 9 | 2-C | 2-C |
| 10 | 2-C | 2-C |
| 11 | 2-C | 2-C |
| 12 | 2-C | 2-C |
| 13 | 2-C | 2-A |
| 14 | 2-C | 2-A |
| 15 | 2-B | 2-B |
| 16 | 2-B | 2-B |
| 17 | 2-C | 2-B |
| 18 | 2-C | 2-B |
| 19 | 2-C | 2-B |
| 20 | 2-A | 2-A |
| 21 | 2-A | 2-A |
| 22 | 2-C | 2-A |
| 23 | 2-C | 2-A |
| 24 | 2-B | 2-A |
| 25 | 2-C | 2-A |

**[Table 5-2]**

| | | |
|---|---|---|
| 26 | 2-B | 2-B |
| 27 | 2-A | 2-A |
| 28 | 2-B | 2-A |
| 29 | 2-A | 2-A |
| 30 | 2-A | 2-A |
| 31 | 2-A | 2-A |
| 32 | 2-A | 2-A |
| 33 | 2-A | 2-A |
| 34 | 2-A | 2-A |
| 35 | 2-A | 2-A |
| 36 | 2-C | 2-B |
| 37 | 2-A | 2-B |
| 38 | 2-C | 2-B |
| 39 | 2-C | 2-B |
| 40 | 2-A | 2-A |
| 41 | 2-A | 2-A |
| 42 | 3-D | 3-C |
| 43 | 3-C | 3-B |
| 44 | 3-C | 3-C |
| 45 | 3-C | 3-C |
| 46 | 3-C | 3-C |
| 47 | 3-C | 3-C |
| 48 | 3-C | 3-C |
| 49 | 3-D | 3-C |
| 50 | 3-D | 3-C |
| 51 | 3-B | 3-B |
| 52 | 3-C | 3-B |
| 53 | 3-B | 3-B |
| 54 | 3-B | 3-B |
| 55 | 3-C | 3-B |
| 56 | 3-C | 3-C |
| 57 | 3-D | 3-C |

### Example 6: Evaluation of VEGFR-2 Agonist Activity Using pERK AlphaLISA Assay

To evaluate the intracellular signaling activation ability of the VEGFR-2 agonist peptides of the present invention, ERK phosphorylation was verified. Using the lysate employed in the pVEGFR AlphaLISA assay, the assay was performed according to the protocol of the AlphaLISA SureFire Ultra p-Erk1/2 (Thr202/Tyr204) Assay Kit (PerkinElmer). Signal detection was performed using a SpectraMax Paradigm multimode microplate reader (Molecular Devices). The obtained signals were analyzed using GraphPad Prism, and % activity was calculated with the maximum signal induced by VEGF165a set as 100% and the unstimulated control as 0%.

The VEGF165a concentration was evaluated at five points (0.08, 0.31, 1.25, 5, 20 nM) using 1/4-fold dilutions starting from 20 nM to determine the maximum value (100%). Peptide concentrations tested were: 3.2, 16, 80 nM for dimer structure number 10, 100 nM for dimer structures numbers 2-41; and 1.25, 5, 20 nM for dimer structures numbers 42-57.

For % activity, peptides showing ≥50% activity at 3.2 nM were designated 1-A, those showing ≥50% activity at 16 nM were designated 1-B, those showing ≥50% activity at 80 nM were designated 1-C, and those showing ≥1% but <50% activity at 80 nM were designated 1-D.

Peptides showing 50% or more activity at 10 nM were designated 2-A, those showing 50% or more activity at 100 nM were designated 2-B, and those showing 1% or more but less than 50% activity at 100 nM were designated 2-C.

Peptides showing 50% or more activity at 1.25 nM were designated 3-A, those showing 50% or more activity at 5 nM were designated 3-B, those showing 50% or more activity at 20 nM were designated 3-C, and those showing 1% or more but less than 50% activity at 20 nM were designated 3-D.

The results are shown in Table 5. Note that none of the samples corresponded to categories 1-A to 1-C, 3-A, or 3-D in the above classification. As shown in Table 5, the synthesized peptide complexes were demonstrated to induce not only phosphorylation of VEGFR-2 but also phosphorylation of ERK, an intracellular signaling molecule.

### Example 7: Evaluation of HUVEC Proliferation Promotion

To evaluate the biological activity of the VEGFR-2 agonist peptide of the present invention, an HUVEC proliferation promotion evaluation was conducted. HUVECs cultured as described above were detached using Tryple (Thermo Fisher Scientific). They were then seeded at 10,000 cells per well onto Nunc^{™} MicroWell^{™} 96 -Well, Nunclon Delta-Treated, Flat-Bottom Microplate (cat. #136101) . After 24 hours, cells were starved by replacing with EBM-2 medium (LONZA) containing 0.5% FBS (Thermo Fisher Scientific), 0.1% Heparin (included with the above Lonza CC-3162), and 0.1% Gentamicin (Nacalai) and cultured for 18 hours. Subsequently, Recombinant Human VEGF 165a Protein (R&D Systems) or the peptide was added, and the cells were cultured for 2 days at 37°C in a CO₂ incubator. Assays were performed according to the CellTiter-Glo (Promega) kit protocol, and signals were detected using a SpectraMax Paradigm multimode microplate reader (Molecular Devices) was used for signal detection. The obtained signals were analyzed using GraphPad Prism, and % activity was calculated with the maximum signal induced by VEGF165a set as 100% and the unstimulated control as 0%.

The VEGF165a concentration was evaluated at six points (0.006, 0.03, 0.16, 0.8, 4, 20 nM) using 1/5-fold dilutions starting from 20 nM to determine the maximum value (100%). For peptide concentrations, dimer structures #2, #5, #13, #14, #25, #27, #29, #31, and #33 were tested at 1, 10, and 100 nM. For dimer structures #42 to #53, tests were conducted at 20 nM and then diluted at 1/4 intervals to four points (0.31, 1.25, 5, and 20 nM).

For % activity, peptides showing 50% or more activity at 1 nM were designated 4-A, those showing ≥50% activity at 10 nM were designated 4-B, those showing ≥50% activity at 100 nM were designated 4-C, and those showing ≥1% but <50% activity at 100 nM were designated 4-D.

Peptides showing ≥50% activity at 0.31 nM were designated 5-A, those with 50% or more activity at 1.25 nM peptide concentration were designated as 5-B, those with 50% or more activity at 5 nM peptide concentration were designated as 5-C, those with 50% or more activity at 20 nM peptide concentration were designated as 5-D, and those with 1% or more but less than 50% activity at 20 nM peptide concentration were designated as 5-E.

The results are shown in Table 6. Note that none of the samples corresponded to categories 4-A, 4-C, or 5-E in the above classification. As shown in Table 6, the synthesized peptide complexes were demonstrated to possess the ability to promote the proliferation of

### HUVEC.

**Table 6. Evaluation of HUVEC Proliferation Promotion**

| [Table 6] | |
|---|---|
| Dimer Structure No. | **proliferation** |
| 2 | 4-D |
| 5 | 4-D |
| 13 | 4-D |
| 14 | 4-D |
| 25 | 4-B |
| 27 | 4-B |
| 29 | 4-B |
| 31 | 4-B |
| 33 | 4-B |
| 42 | 5-B |
| 43 | 5-C |
| 44 | 5-A |
| 45 | 5-C |
| 46 | 5-B |
| 47 | 5-B |
| 48 | 5-A |
| 49 | 5-C |
| 50 | 5-D |
| 51 | 5-C |
| 52 | 5-C |
| 53 | 5-C |

### Example 8: Evaluation of VEGFR Receptor-Specific Activation Activity Using an RTK Array Assay

To evaluate the VEGFR receptor-specific activation activity of the VEGFR-2 agonist peptide of the present invention, phosphorylation of 49 types of tyrosine kinase receptors, including VEGFR-2, was verified.

HUVECs cultured as described above were detached using Tryple (ThermoFisher Scientific), seeded into 6-well plates for adherent cells at a density of 720,000 cells per well, and cultured for 2 days. For starvation, medium was supplemented with 0.5% FBS (ThermoFisher Scientific), 0.1% Heparin (included with the above Lonza CC-3162), and 0.1% Gentamicin (Nacalai) for starvation, and cultured for 18 hours. Subsequently, Recombinant Human VEGF165a Protein (R&D Systems) or peptides were added, and the cells were cultured in a CO₂ incubator at 37°C. After a 10-minute stimulation, cells were lysed using the Lysis Buffer included in the Human Phospho-RTK Array Kit (R&D). Proceeded according to the kit protocol, using C-Digit (Li-COR) for signal detection. The obtained signals were analyzed using the Transparency Overlay Template image included with the kit. VEGF165a was added at a concentration of 5 nM, and the peptides were added at a concentration of 20 nM for evaluation.

The results are shown in Figure 1B. As shown in Figure 1B, the synthesized peptide complexes (dimer structures 48 and 52 in Table 4) exhibited VEGFR-2-specific activation activity similar to that of recombinant VEGF protein.

### INDUSTRIAL APPLICABILITY

This invention may be used in the pharmaceutical and bioengineering industries.

### [Sequence Table]

240823.23-154PCT-seq.xml

## Claims

1. A peptide complex or a pharmaceutically acceptable salt thereof comprising a first peptide and having a VEGFR-2 agonist activity, wherein
the first peptide has an amino acid sequence represented by X¹-W-X²-X³-X⁴-X⁵-X⁶-X⁷-X⁸-Y-X⁹-X¹⁰-X¹¹-C (SEQ ID NO: 1) or consists of an amino acid sequence in which from 1 to 3 amino acids in the amino acid sequence represented by SEQ ID NO: 1 have been substituted, deleted, added, or inserted,
X¹ is an amino acid having in a side chain a chain alkyl group optionally substituted with a polar group or an optionally substituted aromatic ring,
X² is an amino acid having an optionally substituted alkyl group in a side chain,
X³ is an amino acid having an optionally substituted chain alkyl group in a side chain,
X⁴ is an amino acid having an optionally substituted alkyl group in a side chain,
X⁵ is any amino acid,
X⁶ is an amino acid having a chain alkyl group optionally substituted with a polar group in a side chain or is an amino acid that does not have a side chain,
X⁷ is an amino acid having an optionally substituted chain alkyl group in a side chain,
X⁸ is an amino acid having an optionally substituted chain alkyl group in a side chain,
X⁹ is an amino acid having an optionally substituted aromatic ring in a side chain,
X¹⁰ is an amino acid having an optionally substituted alkyl group in a side chain, and
X¹¹ is any amino acid.

2. The peptide complex or the pharmaceutically acceptable salt thereof according to claim 1, wherein
X² is an amino acid having in a side chain a chain or cyclic alkyl group or a chain alkyl group optionally substituted with an aromatic ring,
X³ is an amino acid having a chain alkyl group optionally substituted with a polar group in a side chain,
X⁴ is an amino acid having in a side chain a chain or cyclic alkyl group or a chain alkyl group optionally substituted with a polar group,
X⁷ is an amino acid having in a side chain a chain alkyl group optionally substituted with a polar group or a chain alkyl group optionally branched,
X⁸ is an amino acid having in a side chain a chain alkyl group optionally substituted with a polar group or a chain alkyl group optionally branched, and
X¹⁰ is an amino acid having in a side chain a chain or cyclic alkyl group or a chain alkyl group substituted with an aromatic ring or heterocyclic ring.

3. The peptide complex or the pharmaceutically acceptable salt thereof according to claim 2, wherein
X¹ is W, Hcit, 4Py2NH2, 3Py6NH2, F4aao, or W7N,
X² is V, Tbg, Chg, or Hty,
X³ is D, Q, or MetO2,
X⁴ is V, Tbg, alT, or Chg,
X⁵ is Q, Ahp, W, Hph, Cha, F4COO, or K,
X⁶ is D, G, or N,
X⁷ is D or V,
X⁸ is L, N, or Atb,
X⁹ is F, Bph, Yph, F4G, or F4C,
X¹⁰ is V, Hph, Hty, Chg, or H4Py, and
X¹¹ is D, A, S, F, F4aao, Har, or Hyp.

4. The peptide complex or the pharmaceutically acceptable salt thereof according to claim 1, wherein
X² is an amino acid having a chain or cyclic alkyl group in a side chain,
X⁴ is an amino acid having in a side chain a chain alkyl group optionally substituted with a polar group or a chain alkyl group optionally branched,
X⁶ is an amino acid having a chain alkyl group optionally substituted with a polar group in a side chain,
X⁷ is an amino acid having a chain alkyl group optionally substituted with a polar group in a side chain,
X⁸ is an amino acid having in a side chain a chain alkyl group optionally substituted with a polar group or a chain alkyl group optionally branched, and
X¹⁰ is an amino acid having in a side chain a chain or cyclic alkyl group or a chain alkyl group optionally substituted with an aromatic ring.

5. The peptide complex or the pharmaceutically acceptable salt thereof according to claim 4, wherein
X¹ is W or W7N,
X² is V, Tbg, or Chg,
X³ is D,
X⁴ is V or alT,
X⁵ is Ahp, Cha, or K,
X⁶ is D or N,
X⁷ is D,
X⁸ is L, N, or Atb,
X⁹ is F or F4G,
X¹⁰ is V, Hty, or Chg, and
X¹¹ is D or S.

6. The peptide complex or the pharmaceutically acceptable salt thereof according to claim 1, wherein
the first peptide has an amino acid sequence represented by W-W-V-D-V-Q-D-D-L-Y-F-V-D-C (SEQ ID NO: 2) or consists of an amino acid sequence in which from 1 to 3 amino acids in the amino acid sequence represented by SEQ ID NO: 2 have been substituted, deleted, added, or inserted.

7. The peptide complex or the pharmaceutically acceptable salt thereof according to claim 1, wherein
the first peptide has an amino acid sequence with glycine added to a C-terminus.

8. The peptide complex or the pharmaceutically acceptable salt thereof according to claim 1, wherein
the first peptide is a peptide consisting of an amino acid sequence represented by any one of SEQ ID NOs: 3 to 51.

9. The peptide complex or the pharmaceutically acceptable salt thereof according to claim 1, wherein
the first peptide is a cyclic peptide.

10. The peptide complex or the pharmaceutically acceptable salt thereof according to claim 1, wherein
the first peptide is a cyclic peptide in which an N-terminus amino acid residue is a chloroacetylated amino acid residue, the first peptide has a cysteine residue within the peptide, and the N-terminus amino acid residue and the cysteine residue are bonded to each other.

11. The peptide complex or the pharmaceutically acceptable salt thereof according to claim 1, wherein
the peptide complex includes the first peptide, a second peptide, and a linker connecting the first peptide and the second peptide, and
the second peptide is the same as or different from the first peptide, and has the amino acid sequence represented by SEQ ID NO: 1 or consists of an amino acid sequence in which from 1 to 3 amino acids in the amino acid sequence represented by SEQ ID NO: 1 have been substituted, deleted, added, or inserted.

12. The peptide complex or the pharmaceutically acceptable salt thereof according to claim 11, wherein
a homology between the first peptide and the second peptide is from 90% to 100%, inclusive.

13. The peptide complex or the pharmaceutically acceptable salt thereof according to claim 11, wherein
the first peptide and the second peptide have the same sequence.

14. The peptide complex or the pharmaceutically acceptable salt thereof according to claim 11, wherein
a sixth amino acid of the first peptide and a sixth amino acid of the second peptide, or a C-terminus of the first peptide and a C-terminus of the second peptide, are linked via the linker.

15. The peptide complex or the pharmaceutically acceptable salt thereof according to claim 11, wherein
the linker is a PEG linker or a linker in which 1 to 6 amino acids are added to a PEG linker.

16. A composition comprising the peptide complex or the pharmaceutically acceptable salt thereof according to claim 1 and a carrier.

17. A composition for cell culture that is used to culture cells, the composition comprising the peptide complex or the pharmaceutically acceptable salt thereof according to claim 1 and a carrier.

18. A composition for medical, diagnostic, or research use, comprising the peptide complex or the pharmaceutically acceptable salt thereof according to claim 1 and a carrier.
